# EUROPEAN PATENT SPECIFICATION

(11) **EP 3 784 348 B1**
(45) Date of publication and mention of the grant of the patent: **05.08.2026**
(21) Application number: 19719621.5
(22) Date of filing: 23.04.2019
(51) Int. Cl.: A61K 31/7105, C12N 15/113, A61P 9/00, A61P 17/02, C12N 15/00

(54) **MIR-148B FOR USE IN TREATING DERMAL WOUNDS**
MIR-148B ZUR BEHANDLUNG VON HAUTWUNDEN
MIR-148B POUR LE TRAITEMENT DES PLAIES DERMIQUES

(30) Priority: 23.04.2018 GB 201806569
(43) Date of publication of application: 03.03.2021
(73) Proprietor: Mirava Therapeutics Ltd, Edinburgh, EH10 4AH (GB)
(72) Inventor: CAPORALI, Andrea, Edinburgh Lothian EH16 4TJ (GB); MISCIANINOV, Vladislav, Edinburgh Lothian EH16 4TJ (GB)
(74) Representative: Murgitroyd & Company
(86) International application number: PCT/GB2019/051141
(87) International publication number: WO 2019/207299

(56) References cited:
- EP-A1- 2 545 941
- WO-A1-2013/174692
- WO-A1-2019/043709
- WO-A2-2009/137807
- WANG YU ET AL: "miR-148b-3p functions as a tumor suppressor in GISTs by directly targeting KIT", CELL COMMUNICATION AND SIGNALING, vol. 16, no. 1, 16 April 2018 (2018-04-16), XP093023666, Retrieved from the Internet <URL:http://link.springer.com/content/pdf/10.1186/s12964-018-0228-z.pdf> DOI: 10.1186/s12964-018-0228-z
- AMMAR T. QURESHI ET AL: "Photoactivated miR-148b-nanoparticle conjugates improve closure of critical size mouse calvarial defects", ACTA BIOMATERIALIA, vol. 12, 1 January 2015 (2015-01-01), pages 166 - 173, XP055161491, ISSN: 1742-7061, DOI: 10.1016/j.actbio.2014.10.010
- VLADISLAV MISCIANINOV ET AL: "MicroRNA-148b Targets the TGF-[beta] Pathway to Regulate Angiogenesis and Endothelial-to-Mesenchymal Transition during Skin Wound Healing", MOLECULAR THERAPY, vol. 26, no. 8, 1 August 2018 (2018-08-01), GB, pages 1996 - 2007, XP055595534, ISSN: 1525-0016, DOI: 10.1016/j.ymthe.2018.05.002

## Description

### Field of the Invention

The present invention relates to compositions for the regulation of angiogenesis and endothelial to mesenchymal transition and the use of such compositions and methods in medical treatments such as the treatment of wounds, in particular soft tissue wounds. Accordingly, the compositions and methods described herein can be used to treat conditions and symptoms associated with changes in endothelial-to-mesenchymal transition (EndMT).

### Background to the Invention

It is well appreciated that the TGF-β signalling pathway plays a crucial role in the regulation of both normal and irregular vascular function¹. During canonical activation of the TGF- β pathway, TGF-β binds to the heteromeric receptor complex formed by the activin-like kinase 5 (ALK5, also known as TGF-β type I receptor, TGFBR1) and the TGFβ type II receptor (TGFBR2). Phosphorylation of ALK5 by TGFBR2 kinase, activates its catalytic kinase domain, allowing the activation of the receptor-regulated Smads (i.e. SMAD2 and SMAD3), which translocate to nucleus where they regulate the transcription of specific target genes ².

The activation of TGF-β pathway in endothelial cells (ECs) during angiogenesis is controversial with numerous studies showing that it is either pro-angiogenic or anti-angiogenic in a context-dependent manner. Discrepancies might be explained by the variations in ligand concentrations, culture conditions, cell type and developmental or disease stage *in vivo.* In addition the effects of TGF-β signals depend on the interplay between different members of the TGF-β family ³. Accumulating studies over the past few years have also revealed that activation of TGF-β pathway regulates phenotypic plasticity that is widely displayed by ECs ⁴. One form of EC plasticity is represented by endothelial-to-mesenchymal transition (EndMT), allowing the endothelium to not only alter its fate but to provide a potentially important additional source of mesenchymal cells for participation in fibrosis ^{5 6}. During the EndMT process, ECs lose their endothelial specification and acquire a mesenchymal-like phenotype. Specifically, loss of vascular endothelial cadherin (VE-cadherin) and platelet-endothelial cell adhesion molecule 1 (PECAM-1, CD31) are observed along with elevated expression of α-smooth muscle actin (α-SMA), vimentin, N-cadherin and extracellular matrix (ECM) proteins like collagen type I and III⁷. ECs undergoing EndMT also exhibit cytoskeletal rearrangement, resulting in a change in cell polarity whereby they acquire a stretched and more fibroblast-like morphology ⁷. Recently EndMT has been reported to play an important role during the embryonic stages of cardiac and pulmonary artery development ^{8,9}. In addition, EndMT has a pathological role through promoting tumour growth and progression ¹⁰, contributing to cardiac ¹¹ and renal fibrosis ¹², as well as vascular remodelling ¹³ and atherosclerosis ¹⁴. Furthermore, EndMT has been shown to have an important role in the pathogenesis of scleroderma (Jimenez, 2013, ISRN Rheumatol: 2013:83598).

MicroRNAs (miRNAs) have emerged as post-transcriptional inhibitory regulators of gene expression that bind to complementary messenger RNA (mRNA) transcripts ¹⁵. Their capacity to simultaneously inhibit many different mRNAs allows for efficient amplification of biological responses ¹⁶. Recent studies have revealed important roles for certain miRNAs as therapeutic tools in regulating either physiological or pathological angiogenesis, particularly through the regulation of EC function (reviewed in ^{17,18}). Several miRNAs have been identified that target elements of the TGF- β/Smad signalling axis ¹⁹, and more recently, contribute to the EndMT process ²⁰. Although the role of TGF-β signalling in regulating and maintenance of adult EC homeostasis and plasticity is now well established ², less is known about the mechanisms that regulate the TGF-β signalling pathway. Qureshi et al, Acta Biomaterialia (2015) 12 (1) 166-173 describes the use of photoactivated miR-148b-nanoparticle conjugates to improve closure of critical size mouse calvarial defects. WO2013/174692 A1 describes the use of modulators of miR-148 for the treatment of chronic pulmonary disease. WO2019/043709 A1 describes the use of miRNAs, e.g. miR-148b for the treatment of fibrotic disease, e.g. hepatic disease. WO2009/137807 A2 describes the use of inhibitors of miR-148b for reducing vascularisation. EP 2545941 A1 describes the use of miR-148b for the treatment of heart disease. Wang et al, Cell Communication and Signaling (2018), 16:16 describes the transfection of miR-148-3p into gastrointestinal stromal tumor cells in vitro.

### Summary of the Invention

As described herein, the present inventors have demonstrated that miR-148b targets are strongly enriched for the components of TGF-β pathway. Focusing on the impact of miR-148b on TGF-β signalling, the inventors have demonstrated how this miRNA could regulate EC function and how it impacts on TGF-β to transduce intracellular signals. miR-148b together with miR-148a and miR-152 comprise the miR-148/152 family ²¹. Aberrant expression of miR-148/152 family has been observed in tumours, but also in non-tumour diseases such as IgA nephropathy ²² and atherosclerotic lesions ²³; however, its function has not been explored in ECs.

As shown in the examples, the inventors have surprisingly shown that, in ECs, miR-148b plays a critical role in promoting angiogenesis and that its inhibition promotes EndMT through regulation of the target genes, *TGFB2* and *SMAD2.* Furthermore, they demonstrate that, in an *in vivo* model of wound healing, overexpression of miR-148b increases vascularisation and accelerates wound closure; whereas miR-148b inhibition leads to enhanced wound EndMT and impaired closure.

Accordingly, in a first aspect of the present invention there is provided miR-148b for use in the treatment of a wound.

Wounds, the treatment of which the present invention is particularly suitable, are soft tissue wounds. Specifically, the wound is a dermal wound. While the invention may be used to accelerate wound closure in any type of soft tissue dermal wound, the invention finds particular utility for the treatment of chronic wounds, for example diabetic wounds, such as a diabetic leg ulcer or a diabetic foot ulcer. A diabetic leg ulcer or a diabetic foot ulcer is also an example of a dermal wound. Such wounds are known to be associated with an excess of EndMT.

In the invention, the miR-148b may be natural, e.g. human, miR-148b. The miR-148b for use in the invention may be a functional fragment of a full-length miR-148b. Such functional fragments will comprise a portion of the full-length miR-148b sequence but must retain the ability of the full length miR-148b to accelerate angiogenesis in a wound and/or accelerate wound closure.

Moreover, miR-148b for use in the invention is not limited to natural miR-148b and functional fragments thereof. The miR-148b for use in the invention may be a miR-148b mimic. In another embodiment, the miR-148b for use in the invention may be a pri-miRNA of miR-148b or a pre-miRNA of miR-148b.

In a particular embodiment of the invention, the miR-148b is a miR-148b mimic having the sequence:

CAAGCACGAUUAGCAUUUGAGGUGAAGUUCUGUUAUACACUCAGGCUGUGGCUCUCUGAAA
GUCAGUGCAUCACAGAACUUUGUCUCGAAAGCUUUCUA (SEQ ID NO: 1).

In particular embodiments of the invention, the miR-148b comprises the nucleotide sequence shown as SEQ ID NO: 2:
**AAGUUCUGUUAUACACUCAGGC (SEQ ID NO: 2).**

In particular embodiments of the invention, the miR-148b comprises the nucleotide sequence shown as SEQ ID NO: 3:
**UCAGUGCAUCACAGAACUUUGU (SEQ ID NO: 3).**

SEQ ID NOs: 2 and 3 correspond respectively to the mature hsa-miR-148b-5p and the mature hsa-miR-148b-3p having accession numbers MIMAT0004699 and MIMAT0000759 respectively.

Optionally, the miR-148b for use in the invention comprises a seed region sequence of miR-148b, for example comprises a seed region sequence having the nucleotide sequence shown as SEQ ID NO: 4 or SEQ ID NO:5 :
**CAGUGCA (SEQ ID NO: 4).**
**AGUUCUG (SEQ ID NO: 5).**

SEQ ID NO: 4 and SEQ ID NO: 5 show the nucleotide sequence of the seed sequence of the mature hsa-miR-148b-3p and the mature hsa-miR-148b-5p respectively.

miR-148b for use in the invention may be comprised within a vector, for example a viral vector.

As described in the examples, the inventors in addition to demonstrating that miR-148b enhance wound closure, have also shown that loss of function of miR-148b inhibits wound repair by enabling endothelial to mesenchymal transition in the skin wound model.

Accordingly, in a second aspect of the invention, there is provided an in vitro method of promoting transition of endothelial cells to a mesenchymal phenotype, said method comprising administration of an inhibitor of miR-148b to said endothelial cells, wherein said inhibitor is selected from the group consisting of a LNA anti-miR-148b-3p, synthetic 'Tough Decoy' (TuD) molecule against miR-148b-3p, and a miR-148b-3p sponge.

The demonstration that inhibition of miR-148b may be used to promote transition of endothelial cells to a mesenchymal phenotype enables the use of miR-148b inhibitors to induce collagen production in clinical and in research applications.

Accordingly, in a third aspect of the invention, there is provided an in vitro method of enhancing collagen production in a cell, population of cells, or tissue, said method comprising the administration of a miR-148b inhibitor to said cell, population of cells, or tissue, wherein said inhibitor is selected from the group consisting of a LNA anti-miR-148b-3p, synthetic 'Tough Decoy' (TuD) molecule against miR-148b-3p, and a miR-148b-3p sponge.

The invention demonstrates that miR-148b inhibitors may be of particular use in the treatment of diseases associated with loss of collagen or impaired collagen production. The enhancement of collagen production may be exploited in a therapeutic context, such as in the treatment of arthritis.

In the second and third aspects of the invention, a suitable inhibitor may be, for example, a hsa-miR-148b-3p miRCURY lock nucleic acid (LNA)^{™} miRNA Inhibitor (YI04101119), which is a hsa-miR-148b-3p inhibitor with modified nucleotides to make RNA resistant to degradation, developed by Exiqon/Qiagen. The inhibitor sequence is
CAAAGTTCTGTGATGCACTG **(SEQ ID NO: 6).**

### Brief Description of the Drawings

The invention will now be described further in the following non-limiting examples with reference made to the accompanying drawings in which:

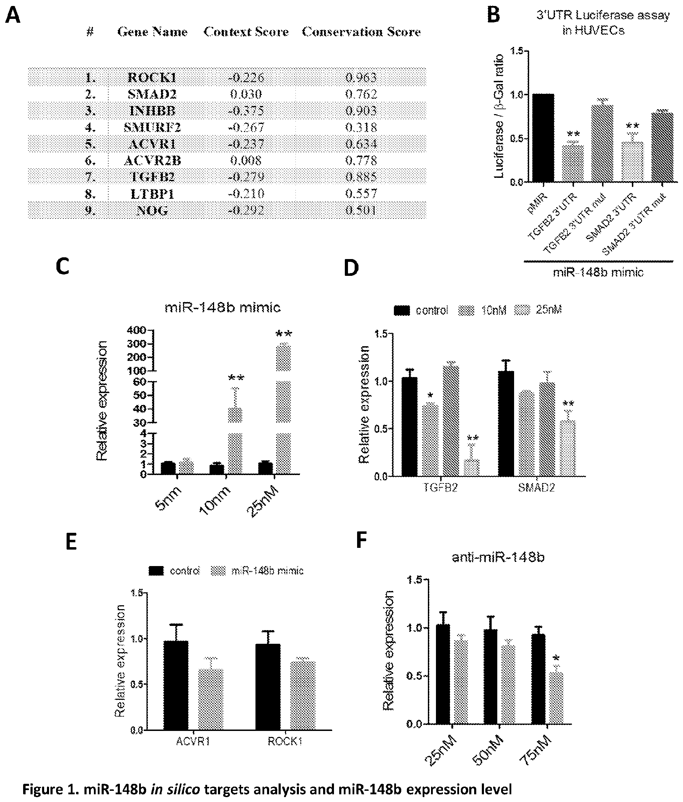

**Figure 1** **illustrates miR-148b *in silico* targets analysis and miR-148b expression level**
   **A.** Analysis of miR-148b target genes using miRpath software. Table shows Context Score and Conservation score from Targetscan. **B.** Luciferase activity at 48h post-co-transfection of HUVECs cells with both miR-148b and the following plasmids: 3'-UTR-TGFB2. 3'-UTR-SMAD2 and pMIR as empty plasmid (n=5); **C.** miR-148b expression levels after miR mimic transfection (n=5); **D.** TGFB2 and SMAD2 expression after different doses of miR-148b mimic transfection (n=5); **E.** ACVR1 and ROCK1 expression miR-148b mimic transfection (n=5); **F.** miR-148b expression levels after anti-miR-148b transfection (n=5). Values are means±SEM. *P<0.05; **P<0.01 vs control. Unpaired two-tailed Student's t-test was applied.

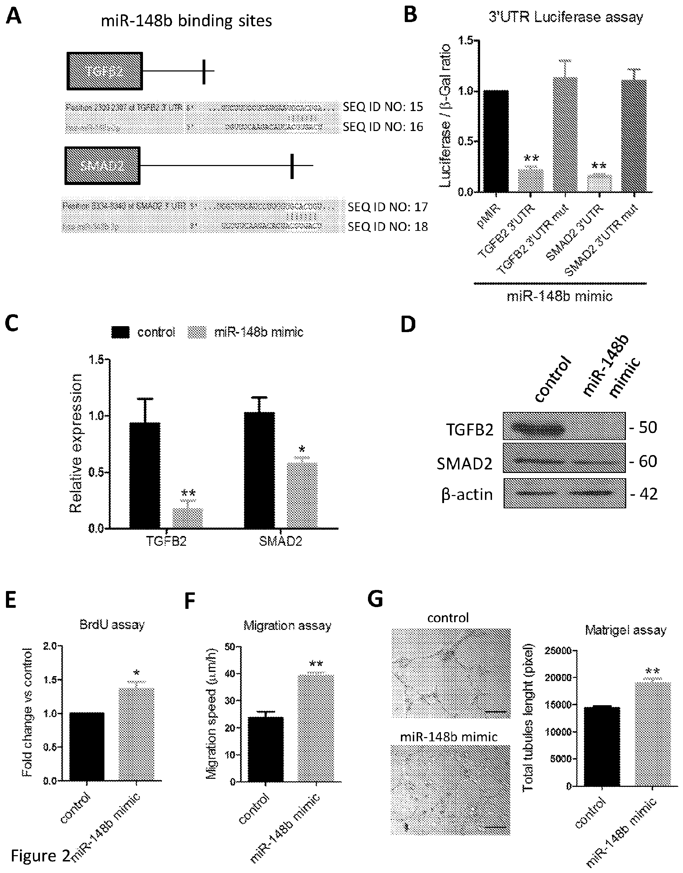

**Figure 2** **illustrates that TGFB2 and SMAD2 are miR-148b target genes:**
   **A**. *In silico* analysis of TGFB2 and SMAD2 3'UTR with Targets can identifies putative miR-148b-binding sites (blue); **B.** Luciferase activity at 48h post-co-transfection of HEK293T cells with both miR-148b and the following plasmids: 3'-UTR-TGFB2. 3'-UTR-SMAD2 and pMIR as empty plasmid (n=5); **C.** Relative gene expression of TGFB2 and SMAD2, after miR-148b overexpression (n=3); **D.** Western blot analysis of TGFB2 and SMAD2 in samples transfected with miR-148b mimic vs control; β-actin is used as a loading control (n=3); HUVECs were transfected with miR-148b mimic or control oligonucleotides for 48hrs; **E.** EC proliferation was analysed by BrdU incorporation assay (n=5); **F.** EC migration reported as migration speed (µm/h) (n=5); **G.** Representative Matrigel assay pictures and quantification as total tubules length (n=5); Values are means±SEM *P<0.05; **P<0.01 vs control and pMIR vector in experiment b. Unpaired two-tailed Student's t-test was applied.

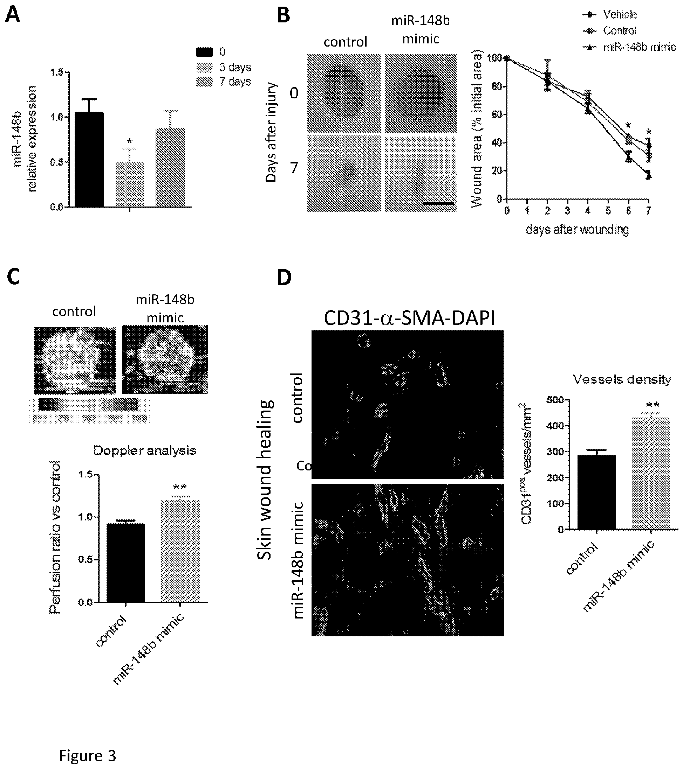

**Figure 3** **illustrates that miR-148b regulates angiogenesis and accelerates wound healing**
   **A.** Expression of miR-148bat 3 and 7 days after wounding. Expression related to not-wounded skin (n=5); **B.** *left*, representative images of control and miR-148b mimic treated wounds at 0 and 7 days post wound injury, scale bar=5mm; *right,* level of wound closure expressed as a percentage of wound area from the initial wound area (n=8); **C.** Representative colour laser Doppler images are taken at 5 days post wounds. Chart shows the level of wound perfusion in mice (calculated as the ratio between treated and control blood flow; n=8 per group); **D.** *left,* Immunohistochemistry for CD31 and α-SMA, in miR-148b mimic-treated or control-oligonucleotide-treated skin wounds; scale bar=100µm (magnification 400x); *right,* quantification of vessel density expressed as CD31 positive vessels/mm² (n=8); Values are means±SEM *P<0.05; **P<0.01 vs control. Unpaired two-tailed Student's t-test was applied.

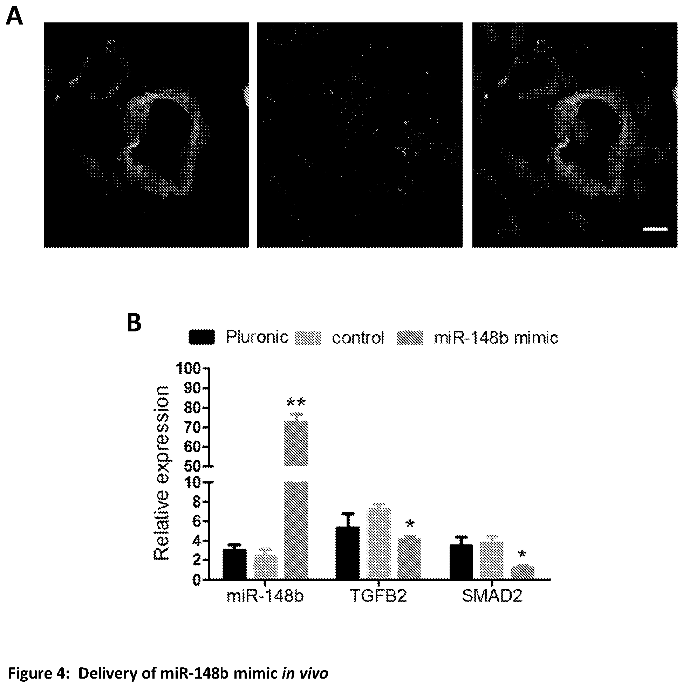

**Figure 4****: Delivery of miR-148b mimic *in vivo***
   A. Representative images of the localization of Cy3 labelled miR-148b (pink dots) in skin wounds. Vessels are stained for CD31 (green), scale bar=25µm (magnification 1000x); **B.** Relative gene expression of miR-148b, *TGFB2* and *SMAD2* in dermal wound after delivering of miR-148b mimic or control oligonucleotides at 7 days (n=5); Values are means±SEM. *P<0.05; **P<0.01 vs control or not-wounded skin. Unpaired two-tailed Student's t-test was applied.

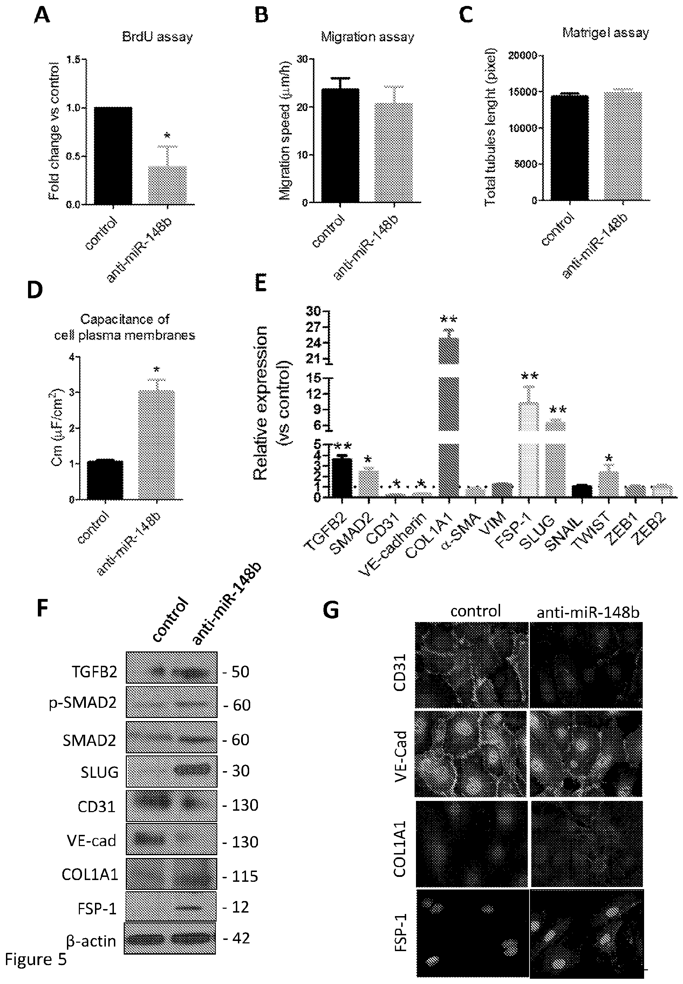

**Figure 5** **illustrates that inhibition of miR-148b induces EndMT.**
   HUVECs were transfected with anti-miR-148b or control oligonucleotides for 48hrs; **A.** EC proliferation was analysed by BrdU incorporation assay (n=5); **B.** EC migration reported as migration speed (µm/h) (n=5); **C.** Matrigel assay quantification as total tubules length (n=5); **D.** Quantification of cell membrane capacitance in HUVECs knock-down for miR-148b or control (n=5); **E.** Relative gene expression of TGFB2, SMAD2, CD31, VE-cadherin COL1A1, SNAIL, SLUG, TWIST, ZEB1/2, VIM, FSP-1 and α-SMA (n=3); **F.** Western blot analysis of TGFB2, SMAD2, p-SMAD2, SLUG, CD31, VE-Cadherin, COL1A1 and FSP-1; β-actin is used as a loading control (n=3); **G.** Immunofluorescence images showing localization of CD31, VE-Cadherin, COL1A1 and FSP-1 in anti-miR-148b or control transfected cells (n=3); scale bar=50µm (magnification 400x). Values are means±SEM. *P<0.05; **P<0.01 vs control. Unpaired two-tailed Student's t-test was applied.

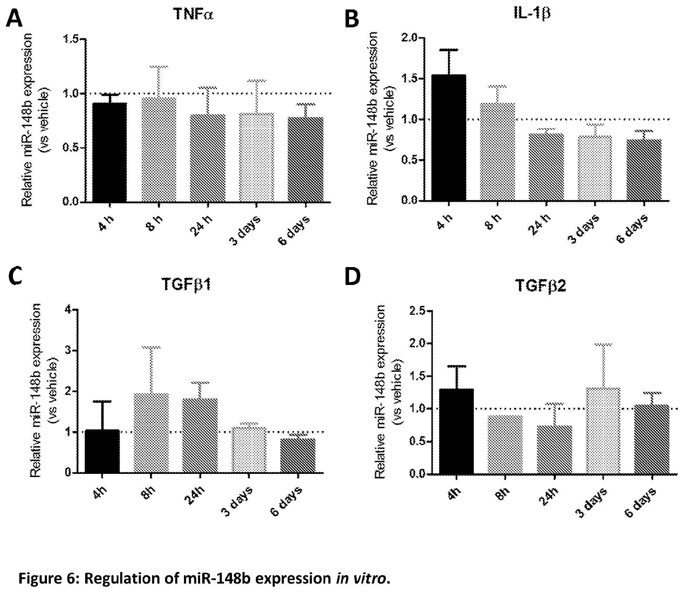

**Figure 6****: Regulation of miR-148b expression *in vitro.*** Relative miR-148b expression after treatment of HUVECs with **A.** TNF-α; **B**. IL-1β; **C**. TGFβ1; **D**. TGFβ2; the dotted line represents relative miR-148b expression in the untreated control HUVEC (n=3); Values are means±SEM.

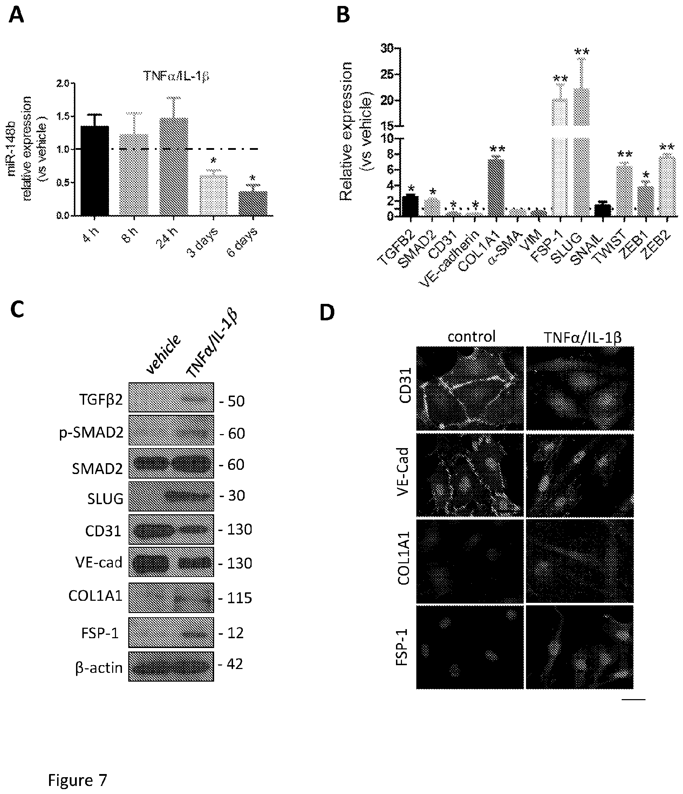

**Figure 7** **illustrates that chronic inflammation downregulates miR-148b in ECs** HUVECs were treated with a combination of TNF-α and IL-1β for 6 days. **A.** miR-148b expression levels after TNF-α/IL-1β treatment; **B**. Relative expression of miR-148b, TGFB2, SMAD2, CD31, VE-cadherin, SNAIL, SLUG, TWIST, ZEB1/2, VIM, FSP-1, α-SMA and COL1A1; C. Western blot analysis of TGFB2, p-SMAD2, SMAD2, CD31, VE-Cadherin, SLUG, FSP-1 and COL1A1; β-actin was used as a loading control; **D.** Immunofluorescence images showing localization of CD31, VE-Cadherin, COL1A1 and FSP-1 in TNF-α/IL-1β treated cells; scale bar=50µm (magnification 400x). Values are means±SEM. *P<0.05; **P<0.01 vs vehicle. Unpaired two-tailed Student's t-test was applied.

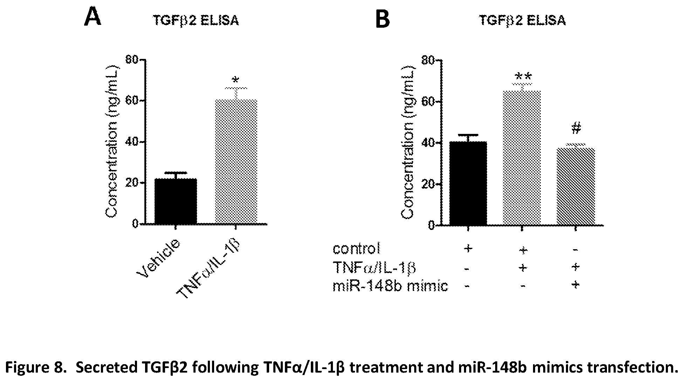

**Figure 8****. Secreted TGFβ2 following TNFα/IL-1β treatment and miR-148b mimics transfection. A.** Quantification of TGFβ2 ELISA experiment expressed in TGFβ2 concentration (ng/mL) in TNF- α/IL-1β treated samples vs the control; **B.** Quantification of TGFβ2 ELISA experiment expressed in TGFβ2 concentration (ng/mL) in miR-148b gain-of-function and/or TNFα/IL-1β treated samples vs the control; Values are means±SEM (n=5). *P<0.05; **P<0.01 vs control; #P<0.05 vs TNFα/IL-1β. Unpaired two-tailed Student's t-test and one-way ANOVA statistical test followed by Bonferroni post-hoc analyses were applied.

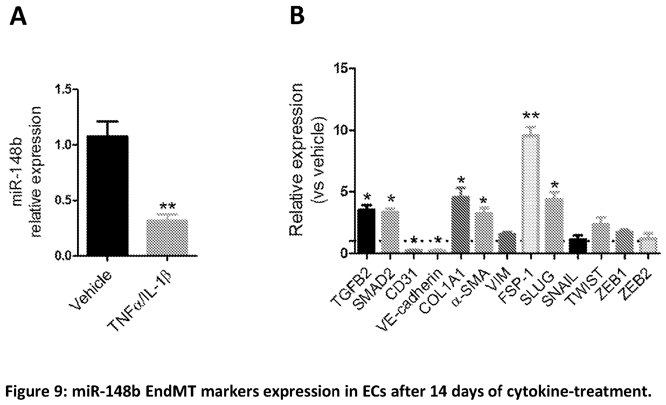

**Figure 9****: miR-148b EndMT markers expression in ECs after 14 days of cytokine-treatment. A.** Relative miR-148b expression after treatment of HUVECs treated with TNFα/IL-1β for 14 days; **B.** Relative expression of TGFB2, SMAD2, CD31, VE-cadherin, SNAIL, SLUG, TWIST, ZEB1/2, VIM, FSP-1, α-SMA and COL1A1; the dotted line represents relative expression in the vehicle-treated HUVEC (n=3); Values are means±SEM. *P<0.05; **P<0.01 vs vehicle. Unpaired two-tailed Student's t-test was applied.

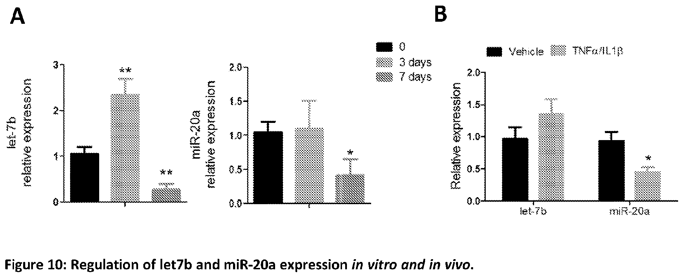

**Figure 10****: Regulation of let7b and miR-20a expression *in vitro and in vivo.* A.** Relative let7-b and miR-20a expression in vivo during wound healing progression and **B.** following TNF-α/IL-1β treatment in ECs Values are means±SEM (n=3). *P<0.05; **P<0.01 vs day 0 . *P<0.05 vs vehicle. Unpaired two-tailed Student's t-test was applied.

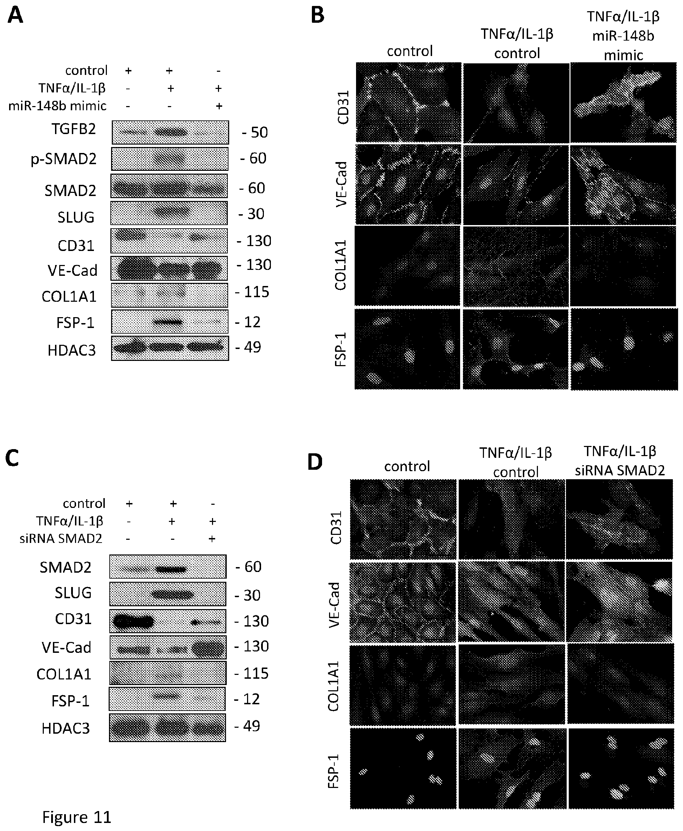

**Figure 11** **illustrates that overexpression of miR-148b rescues cytokines-induced EndMT** HUVECs were transfected with miR-148b mimic, SMAD2 siRNA or control and treated with TNF-α/IL-1β for 6 days. **A.** Representative Western blot analysis of TGFB2, SMAD2, p-SMAD2 CD31, VE-Cadherin, SLUG, FSP-1 and COL1A1 in; HDAC3 is used as a loading control (n=3); **B.** Immunofluorescence images showing localization of CD31, VE-Cadherin, COL1A1 and FSP-1 in miR-148b or control transfected HUVECS and/or treated with TNF-α/IL-1β; scale bar=50µm (magnification 400x) (n=3). **C.** Representative Western blot analysis of SMAD2, CD31, VE-Cadherin, SLUG, COL1A1 and FSP-1; HDAC3 is used as a loading control (n=3). **D.** Immunofluorescence images showing localization of CD31, VE-Cadherin, COL1A1 and FSP-1 in SMAD2 siRNA or control transfected HUVECS and/or treated with TNF-α/IL-1β; scale bar=50µm (magnification 400x) (n=3).

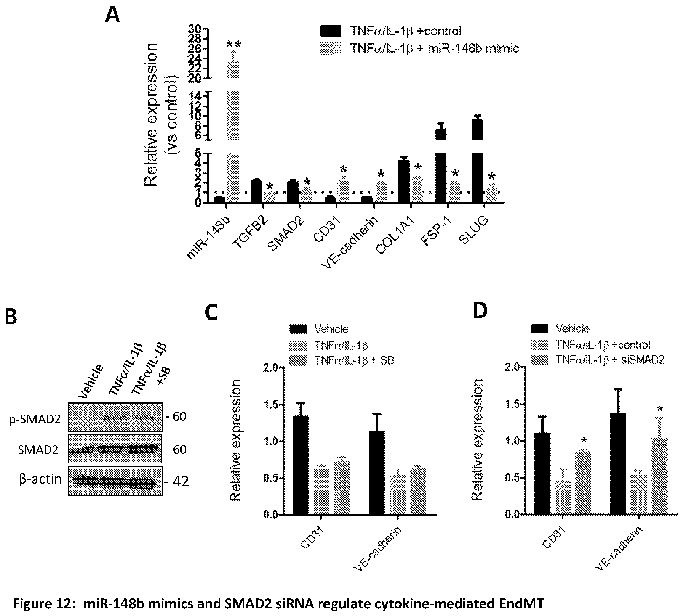

**Figure 12****: miR-148b mimics and SMAD2 siRNA regulate cytokine-mediated EndMT** HUVECs were transfected with miR-148b mimic, SMAD2 siRNA or control and treated with TNF-α/IL-1β for 6 days. **A.** Relative expression of miR-148b, TGFB2, SMAD2, CD31, VE-cadherin, COL1A1, FSP-1 and SLUG (n=3); **B.** Western blot for phospho-SMAD2, SMAD2 and β-actin in HUVECs treated with TNF-α/IL- 1β and ALK5 inhibitor (SB431542; 10mM) for 6 days. **C.** Relative expression CD31 and VE-Cadherin in HUVECs following treatment with ALK5 inhibitor or **D.** SMAD2 siRNA and/or TNFα/IL-1β ; Values are means±SEM (n=3). *P<0.05; **P<0.01 vs TNFα/IL1β + control oligonucleotides. Unpaired two-tailed Student's t-test was applied.

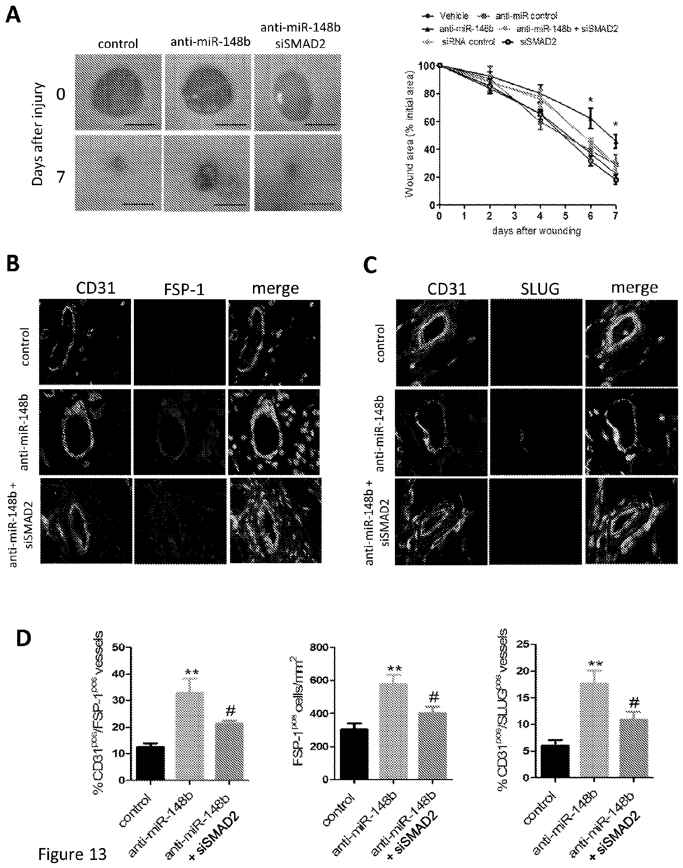

**Figure 13** **illustrates that inhibition of miR-148b delays wound closure and promotes EndMT in a model of wound healing**
   Dermal wounds were treated with anti-miR control or siRNA control oligonucleotides and anti-miR-148b and/or SMAD2 siRNA for 7 days. **A.** *left*, Representative images of treated wounds at 0 and 7 days post wound injury, scale bar=5mm; *right,* level of wound closure expressed in percentage of wound area from the initial wound area (n=8); **B.** Immunohistochemistry localization of CD31 (green) and FSP-1 (red) in the wound vessels; scale bar=25µm (magnification 630x) (n=8), nuclei are stained with DAPI (blue); **C.** Immunohistochemistry localization of CD31 and SLUG in the wound vessels; scale bar=25µm (magnification 630x) (n=8), nuclei are stained with DAPI (blue); **D.** Quantification of CD31/FSP-1 double positive vessels and FSP-1 positive cells and CD31/SLUG double positive vessels in the wounds; n=8 per each group. Values are means±SEM. *P<0.05; **P<0.01 vs anti-miR control. ^{#}P<0.05 vs anti-miR-148b. Unpaired two-tailed Student's t-test and one-way ANOVA statistical test followed by Bonferroni post-hoc analyses were applied.

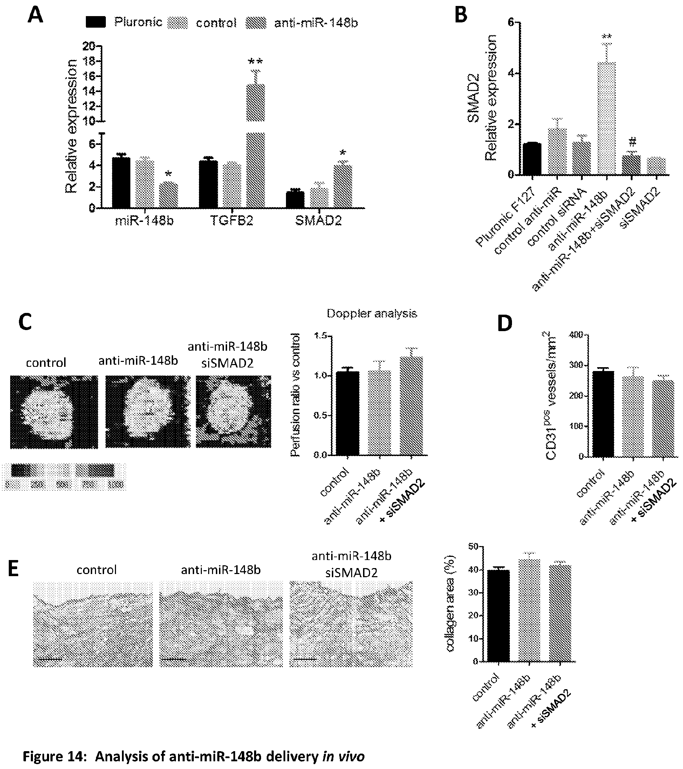

**Figure 14****: Analysis of anti-miR-148b delivery *in vivo***
   Dermal wounds were treated with control and anti-miR-148b or SMAD2 siRNA at 7 days. **A.** Relative expression of miR-148b, TGFB2 and SMAD2 (n=5); **B.** Relative SMAD2 expression in dermal wounds treated (n=5); **C**. *left* Representative colour laser Doppler images are taken at 5 days post wounds. *Right.* Chart shows level of wounds perfusion in mice (calculated as the ratio between treated and control blood flow; n=8 per group). **D.** Quantification of vessel density expressed as CD31 positive vessels/mm2 (n=8); **E**. *left,* Sirius Red staining of wounds obtained at day 7 post wounding; *right,* Collagen quantification. Scale bar represents 100µm (magnification 200x). Values are means±SEM. *P<0.05; **P<0.01 vs control; #P<0.05 vs anti-miR-148b. Unpaired two-tailed Student's t-test and one-way ANOVA statistical test followed by Bonferroni post-hoc analyses were applied.

### Detailed Description

As described above, the present inventors have shown that miR-148b may be used to enhance wound healing.

In the invention, any suitable miR-148b may be used.

miRNA genes are typically transcribed by RNA polymerase II(Pol II), although some are transcribed by Pol III. Animal miRNAs are initially transcribed as part of a miRNA precursor, primary miRNA (pri-miRNA) of several hundred nucleotides. A single pri-miRNA may comprise from one to six miRNA precursors, each of which comprises a hairpin loop structure of about 70 nucleotides.

### miRNA

The double-stranded RNA structure of the hairpins in a pri-miRNA is recognised by the nucleic protein DGCR8 which associates with the enzyme Drosha to form the Microprocessor complex. DGCR8 orientates the catalytic domain of Drosha to cleave RNA approximately 11 nucleotides from the hairpin base and thus liberate hairpins from the pri-miRNAs. The resulting pre-miRNA has a 2-nucleotide overhang at its 3' end. In certain species, pre-miRNAs may be spliced directly into introns without using the Microprocessor complex.

Pre-miRNA hairpins are exported from the nucleus into the cytoplasm, where the pre-miRNA hairpin is cleaved by Dicer. Dicer removes the loop structure of the pre-miRNA hairpin yielding a miRNA; miRNA * duplex of approximately 22 nucleotides in length. The two strands of the duplex are then separated from each other by the Dicer-TRBP complex, with one strand incorporated into the RNA induced silencing complex (RISC) where miRNA and its mRNA target interact. RISC, the strand incorporated into RISC is the mature miRNA. The other strand is called miRNA* strand and is usually degraded. However, sometimes, both strands may function as mature miRNAs. To fulfil the function in gene regulation, an miRNA must be complementary to a part of one or more mRNAs. For partially complementary micro RNAs to recognise their targets, nucleotides 2-7 of the miRNA, known as the "seed region", must be perfectly complementary. The mature miRNA guides RISC to a target site within mRNA, where, if there is perfect complementarity to the mature miRNA, the mRNA is cleaved. If the target site has lower complementarity to the mature miRNA, the mRNA is not cleaved. However, translation of the mRNA is typically suppressed.

In the present invention, the miR-148b may be a mature miRNA. Alternatively, miR-148b for use in the invention may be a pri-miRNA 148-b molecule or a pre-miRNA 148-b molecule. miR-148b for use in the invention may be a natural miRNA.

For example, it may be a human miR-148b.

In a particular embodiment of the invention, the miR-148b is a miR-148b mimic having the sequence:

CAAGCACGAUUAGCAUUUGAGGUGAAGUUCUGUUAUACACUCAGGCUGUGGCUCUCUGAAA
GUCAGUGCAUCACAGAACUUUGUCUCGAAAGCUUUCUA (SEQ ID NO: 1).

SEQ ID NO: 1 corresponds to the sequence for the human precursor miR-148b molecule (Accession No: MI0000811).

In particular embodiments of the invention, the miR-148b comprises the nucleotide sequence shown as SEQ ID NO: 2:
**AAGUUCUGUUAUACACUCAGGC (SEQ ID NO: 2)**.

In particular embodiments of the invention, the miR-148b comprises the nucleotide sequence shown as SEQ ID NO: 3:
**UCAGUGCAUCACAGAACUUUGU (SEQ ID NO: 3).**

SEQ ID NOs: 2 and 3 correspond respectively to the mature hsa-miR-148b-5p and the mature hsa-miR-148b-3p having accession numbers MIMAT0004699 and MIMAT0000759 respectively.

Other miR-148b molecules which may be used in the invention include, for example, mouse miR-148b molecules, for example as having Accession Number MI0000550, or rat miR-148b molecules, such as having Accession Number MI0000616.

Moreover, the miR-148b for use in the invention is not limited to naturally derived miRNA 148-b, either from human or other animal genomes. miR-148b for use in the invention may be an miRNA 148-b mimic, which generates a biologically equivalent effect to a naturally derived miRNA 148-b, for example one or more of the ability to reduce expression of *TGFB2* and/or *SMAD2,* (as measured for example by means of a luciferase assay), to accelerate wound closure, to increase wound perfusion and/or to increase vessel density in a wound.

Any suitable miRNA 148-b mimic may be used. For example, in one embodiment, said miRNA mimic may comprise a miRNA sequence containing the same seed region as the human miR-148b molecule. Optionally, the miR-148b for use in the invention comprises a seed region sequence having the nucleotide sequence shown as SEQ ID NO: 4 or SEQ ID NO: 5. The length of the miRNA mimic may be reduced compared to the natural miR-148b sequence such that a short-length miRNA mimic consisting of approximately 14-17 nucleotides may be used. The miR-148b mimic may be modified compared to the natural miR-148b at the base moiety, sugar moiety, or phosphate backbone. Such modifications may improve, for example, hybridisation or stability of the molecule. For example, miRNA mimics may partially comprise a phosphorothioate structure in which the RNA phosphate backbone structure is substituted with another element such as sulphur. Other modifications which can be made to a miRNA mimic compared to natural miRNA include the substitution of the RNA with DNA, PNA (peptide nucleic acid) or LNA (locked nucleic acid). Furthermore, the 2' hydroxyl group of RNA sugar may be modified by, for example, methylation, methoxylation, fluorination, etc.

In a particular embodiment, the miR-148b molecule for use in the invention is a miR-148b mimic having the nucleotide sequence shown as SEQ ID NO: 1.

Typically, while miRNA which is active *in vivo* is single-stranded, for delivery, it is preferred to deliver the miRNA molecule in a double-stranded form to enable binding to RISC. Thus, in a preferred embodiment, the miR-148b for use in the invention is double-stranded. However, in other embodiments, the miR-148b molecule may be introduced in the form of a single-stranded RNA molecule or an RNA molecule which is at least partially double-stranded.

### Treatment

As described herein, the inventors have shown that miR-148b may be used to inhibit endothelial-to-mesenchymal transition (EndMT). EndMT has been previously shown to have a detrimental influence on skin wound healing by, for example, resulting in fibrosis and excessive collagen deposition. As shown in the examples, the inventors have shown that miR-148b accelerates wound closure. The invention may therefore find particular use in the treatment of wounds, in particular soft tissue wounds. In the context of the present invention, a "wound" refers to an injury to any soft tissue, including acute, sub-acute, and "non-healing" wounds, such as chronic wounds.

The present invention may find use in the treatment of wounds such as, but not limited to, lacerations, incisions, abrasions, penetrating wounds, surgical wounds, hematomas, contusions, burns, chronic wounds, such as diabetic wounds, and ulcers.

The invention would be of particular use in the treatment of "non-healing wounds", i.e. wounds that exhibit impaired healing, such as delayed acute wounds and chronic wounds. Such wounds are associated with enhanced endothelial-to-mesenchymal transition (EndMT) In one embodiment, the invention may be for use in the treatment of non-healing wounds such as chronic wounds associated with diabetes, ischemia, venous stasis disease, or pressure. For example, lower leg and foot ulcers are particularly prevalent in diabetic patients. In one particular embodiment, the invention may be used in the treatment of chronic wounds associated with diabetes, such as diabetic leg or foot ulcers.

In the context of the present invention, treatment of a wound may include one or more of reducing swelling, reducing inflammation, and/or reducing scar formation associated with a wound. Treatment may thus include curative effects, acceleration of healing effects, or alleviation of symptoms.

### Pharmaceutical Compositions

The miR-148b molecules of and for use in the invention may be administered as a pharmaceutical composition. Pharmaceutical compositions according to the present invention, and for use in accordance with the present invention may comprise, in addition to active ingredients, a pharmaceutically acceptable excipient, a carrier, buffer, stabiliser or other materials well known to those skilled in the art (see, for example, Remington: the Science and Practice of Pharmacy, 21st edition, Gennaro AR, et al, eds., Lippincott Williams & Wilkins, 2005). Such materials may include buffers such as acetate, Tris, phosphate, citrate, and other organic acids; antioxidants; preservatives; proteins, such as serum albumin, gelatin, or immunoglobulins; hydrophilic polymers such as polyvinylpyrrolidone; amino acids such as glycine, glutamine, asparagine, histidine, arginine, or lysine; carbohydrates; chelating agents; tonicifiers; and surfactants. In one embodiment, the pharmaceutical composition comprises a block copolymer, for example a block copolymer which demonstrates reverse thermal gelation behavior such that at body temperature said copolymer is solid whereas at a lower temperature such as at 4 C such copolymers are solid. An example of such a copolymer which may be used to deliver a miR-148b molecule is a polyoxthylene - polyoxypropylene block co-polymer such as Pluronic F-127 gel.

The pharmaceutical compositions may also contain one or more further active compounds selected as necessary for the particular indication being treated, preferably with complementary activities that do not adversely affect the activity of the compound or composition of the invention. For example, for the treatment of wounds, the miR-148b molecules as described in the present application may be combined with other therapeutic agents such as anti-inflammatories, antibiotics, antimicrobial drugs etc.

The active ingredients may be administered via microspheres, microcapsules, liposomes, other microparticulate delivery systems. For example, active ingredients may be entrapped within microcapsules which may be prepared, for example, by coacervation techniques or by interfacial polymerization, for example, hydroxymethylcellulose or gelatin microcapsules and poly- (methylmethacylate) microcapsules, respectively, in colloidal drug delivery systems (for example, liposomes, albumin microspheres, microemulsions, nano-particles and nanocapsules) or in macroemulsions. For further details, see Remington: the Science and Practice of Pharmacy, 21st edition, Gennaro AR, et al, eds., Lippincott Williams & Wilkins, 2005.

### Vectors

miR-148b molecules for use in the invention may be delivered via an expression system utilising a vector . The vector can include a promoter operably linked to nucleic acid encoding the miR-148b nucleic acid. For example, the vectors which may be used include viral and non-viral vectors, such as AAV serotypes, adenovirus, herpes virus, SV40, HIV, SIV and other lentiviral vectors, RSV and non-viral vectors including naked DNA, plasmid vectors, peptide-guided gene delivery, terplex gene delivery systems, calcium phosphate nanoparticles, magnetic nanoparticles, colloidal microgels and/or the integrase system from bacteriophage phiC31. Cationic lipid-based non-viral vectors can include glycerol-based (e.g. DOTMA, DOTAP, DMRIE, DOSPA), non-glycerol-based (e.g. DOGS, DOTIM) and/or cholesterol-based cationic lipids (e.g. BGTC, CTAP; Ju et al.(2015). Int. J. Mol. Sci. 16, 5666-5681), 2015; Karmali and Chaudhuri, (2007). Med. Res. Rev. 27, 696-722.; Lee et al., (2016) Theranostics 6, 192-203.). Viral and non-viral vector delivery may be accompanied by other molecules such as cationic lipids and/or polymers and/or detergents and/or agents to alter pH, such as, for example, polyethelene glycol (PEG), to enhance cellular uptake of vectors and/or to enhance expression from vectors and/or to evade the immune system. For example, polycationic molecules have been generated to facilitate gene delivery including but not exclusive to cationic lipids, poly-amino acids, cationic block co-polymers, cyclodextrins amongst others. Pegylation of vectors with polyethelene glycol (PEG) can shield vectors from, for example, the extracellular environment. Vectors may be used in conjunction with agents to avoid or minimise cellular immune responses such as PEG or as a Polyplex with Poly (L-Lysine) among others. Vectors may be delivered in conjunction with immunomodulatory / immunosuppression regimes to aid transgene expression. Vector delivery may be undertaken using physical methodologies such as electroporation, nucleofection and/or ionotophoresis, either alone or in combination with molecules to enhance delivery. Vectors may be used in conjunction with agents to promote expression of transgenes incorporated into vectors, for example, using histone deacetylase inhibitors (HDAC) and/or DNA methyl transferase inhibitors and/or histone methyl transferase inhibitors to modulate chromatin structures thereby aiding expression. HDAC inhibitors include but are not exclusive to short chain fatty acids such as valproic acid and sodium butyrate, ketones, benzamides, cyclic and non-cyclic hydroxamates such as suberoyl anilide hydroxamic acids (SAHA), trichostatin A (TSA), cyclic peptides or tetrapeptides amongst others (Daly et al., (2016) Histone deacetylase: Therapeutic targets in retinal degeneration. doi:10.1007/978-3-319-17121-0_61; Liu et al., (2006) Cancer Treat Rev 32, 157-165. doi:10.1016/j.ctrv.2005.12.006; Ververis et al., (2013) Biol. Targets Ther. 7, 47-60). DNA methyl transfease inhibitors including, for example, 5-AC, decitabine and zebularine can be used to modulate chromatin structures. In addition, histone methyl transferase inhibitors can influence chromatin states, for example, BIX-01294 (diazepin-quinazolin-amine derivative).

### Examples

### Materials and Methods

### Cells and Cell Culture

Human umbilical vein ECs (HUVECs; Lonza) were grown in EGM-2 (comprising EBM-2 with growth factors and other supplements) with 2% Foetal Bovine Serum. Confluent HUVECs were treated with 10 ng/ml TNF-α, IL-1β, TGFβ2 (PeproTech); medium and cytokines were changed every two days. SB431542 (Tocris) has been used at the dose of 10µM. HEK293T cells (*ATCC^{®},* CRL-11268) were cultured in D-MEM with 10% FBS (Life Technologies).

### miR-148b Target Analysis

Computational prediction of miR-148b target genes was done using published algorithm TargetScan (www.targetscan.org). miRpath v.3 (http://diana.imis.athena-innovation.gr/DianaTools/index.php?r=mirpath) was used to perform gene set enriched analysis of miR-148b target genes. The prediction is based on Targetscan parameters such as Context score and Conservation score. The Context score for a specific site is the sum of the contribution of 14 features of the 3' UTR structure ⁵⁰; the Conservation score is the probability of conserved targeting ⁵¹. TargetScan considers matches to human 3' UTRs and their orthologs, as defined by UCSC whole-genome alignments. Lowest Context score shows a more favourable binding. Higher conservation score represent a broad conservation of the binding sites between the species.

### RNA Extraction and Quantitative Real-Time Analysis

Total RNA was extracted using miReasy kit (Qiagen). Real-time quantification to measure miRNAs was performed with the TaqMan miRNA reverse transcription kit and miRNA assay (Life Technology) using Lightcycler 480 (Roche). miRNAs expression was normalized to the U6 small nucleolar RNA (snRU6). For mRNA analysis, cDNA was amplified by quantitative real-time PCR (qPCR) and normalized to 18S ribosomal RNA. Each reaction was performed in triplicate. Quantification was performed by the 2^{-ΔΔCt} method ⁵². qPCR was used to measure the expression of miR-148b (miR-148b-3p Thermo Fisher Scientific, Cat. #4426961), snRU6 (Thermo Fisher Scientific, Cat. #4331182), TGFβ2, SMAD2, CD31, VE-Cadherin, COL1A1, α-SMA, SLUG, VIM, SNAIL, TWIST, ZEB1/2 and 18S rRNA. Primers are pre-designed from Sigma (KiCqStart^{™} Primers).

### Cells transfection, transduction and functional assays

Lipofectamine RNAiMAX (Thermo Fisher Scientific) was used to transfect HUVECs with miR-148b mimic, anti-miR-148b and mimic or anti-miR control (25nM and 75mM, respectively final concentration) or with siRNA targeting SMAD2 (20nM final concentration), according to the manufacturer's instructions.jetPEI-HUVECs (Polyplus) was used to transfect HUVECs with DNA plasmids. The following functional assays were performed: BrdU incorporation assay using Cell Proliferation colorimetric assay (Roche); Matrigel assay with HUVECs was performed as previously described using BD Matrigel Basement Membrane Matrix (BD Biosciences) ⁵³.

### ECIS Assays: migration assay and plasma membrane capacitance analysis

Confluent HUVECs were transfected with controls or miR-148b mimic or anti-miR-148b and, plated on the ECIS chip array (8W1E or 8W10E). The migration speed was calculated in µm/h, capacitance of the plasma membrane is Cm (µF/cm²) as reported in Giaever, I, and Keese, CR (1991) ²⁹.

### Western-Blot analyses

Proteins were extracted from cultured cells or muscles by using ice-cold buffer A (50mM Hepes, 150mM NaCl, 1mM EDTA, 1mM EGTA, 25mM NaF, 5mM NaPPi, 1%Triton, 1% NP40, 1 mM Na3VO4, 0,25% sodium deoxycholate, 0.5mM Na-orthovanadate, 1 mM benzamidine, 0.1mM phenylmethylsulfonyl fluoride). Protein concentration was determined using the Bio-Rad Protein Assay Reagent (Bio-Rad Laboratories, UK). Detection of proteins by Western blot analyses was done following separation of whole cell extracts (20µg) on SDS-polyacrylamide gels. Proteins were transferred to nitrocellulose membranes and probed with the following antibodies: TGFβ2 (Abcam, ab36495, 1:1000), SMAD2 (SantaCruz Biotechnology, Santa Cruz, CA, USA; #3102, 1:1000), p-SMAD2 (465/467) (Cell Signalling; #3108, 1:1000); CD31 (Abcam., 1:1000), VE-Cadherin (Abcam, ab33168, 1:1000), COL1A1 (Abcam, ab138492, 1:200), FSP-1 (S100A4; Abcam, ab41532, 1:1000), SLUG (Abcam, ab27568, 1:1000), β-actin (Abcam, ab16039, 1:4000), HDAC3 (GeneTex GTX113303, 1:1000) and GAPDH (GeneTex, GT239, 1:1000) ( both used as loading control). For detection, the inventors used secondary antibodies conjugated to horseradish peroxidase which were rabbit anti-mouse (Abcam, ab97046, 1:5000) and goat anti-rabbit (Abcam, ab6721, 1:10000). Detection was developed by chemiluminescence reaction (ECL) (Immunoblot, Millipore, 23225).

### Immunofluorescence

Cells were fixed using 4% paraformaldehyde in PBS at room temperature for 15 min. For intracellular staining, fixed cells were permeabilized using 0.5% Triton X-100 in PBS (Sigma-Aldrich) or 0.1% Saponin in PBS at room temperature for 10 min. The blocking of specific antibody activity was performed using 3% bovine serum albumin (BSA) in PBS for 1 hour. Samples were incubated with antibodies to CD31 (Abcam, ab28364, 1:50), VE-cadherin (Abcam, ab33168, 1:100), FSP-1 (Abcam, ab41532, 1:200) and COL1A1 (Abcam, ab138492, 1:100) in PBS containing 1% BSA at 4°C overnight. Samples were washed extensively with PBS and incubated with Alexa-Fluor-488-conjugated antibodies to rabbit IgG (Life Technologies, Carlsbad, CA, #A11070) and Alexa-Fluor-594-conjugated antibodies to rabbit IgG (Life Technologies, Carlsbad, CA, #A11072) in DAPI with PBS with 1% BSA at room temperature for 1 h. Image analysis was performed on Zeiss LSM 780 confocal microscope.

### Luciferase assay

Luciferase assay has been performed as previously described ⁵³. TGFB2 3'-UTR and SMAD2 3'-UTR vectors were cloned in pMIR-Reporter (Life Technology). Primers are used for the cloning are: TGFβ2 3'UTR: Forward 5'-ATAAAGCTTATTTGCCACATCATTGCAGA-3' (SEQ ID NO: 7), Reverse 5'-ATAACTAGTGGGAATAAAAAGACGGCACA-3' (SEQ ID NO: 8); SMAD2 3'UTR: Forward 5'-ATAAAGCTTTGATCCAGCTAAGGTAACTGATGTT-3' (SEQ ID NO: 9), Reverse 5'-ATAACTAGTTGGTAAACAACTCAAATGGCTTTC-3' (SEQ ID NO: 10). Primers for 3'UTR mutation: TGFB2: Forward ATAAAGCTTATTTGCCACATCATTGCAGA (SEQ ID NO: 11) and Reverse ATAACTAGTCCTATCTGAGAGGAAAATGTCTGC (SEQ ID NO: 12); SMAD2 Forward ATAAAGCTTTGATCCAGCTAAGGTAACTGATGTT (SEQ ID NO: 13) and Reverse ATAACTAGTGGACTTCCAGAGGGAAACAA (SEQ ID NO: 14). Luciferase constructs were transfected into HEK293T cells or HUVECs together with miR-148b mimics or p-SV-beta -Gal control vector. Cells were cultured for 48h and assayed with the Luciferase and β-Galactosidase Reporter Assay Systems (Promega). Luciferase values were normalized to protein concentration and β-galactosidase activity.

### In vivo experiments

All experiments involving mice were performed in accordance with the guidance and the operation of Animals (Scientific Procedures) Act 1986 and prior approval of the UK Home Office and the University of Edinburgh ethic committee. Skin wound healing protocol has been performed as described before ⁵⁴⁻⁵⁶. CD-1 female mice (7-9-week-old) (n=8 per group) were randomly assigned a treatment group and anaesthetized with isofluorane. Two full-thickness excisional wounds were made to the shaved dorsal skin. Full-thickness excisional wounds were made by picking up a fold of skin and using a sterile, disposable 5-mm biopsy punch (Kai Industries), resulting in generation of one wound on each side of the midline. Control oligonucleotides (scramble sequence), miR-148b mimic, Cy3-labeled miR-148b mimic or anti-miR-148b or SMAD2 siRNA (1µg/wound) were delivered topically by pipette into the wound cavity (10µl in a vehicle of 30% Pluronic F-127 gel [is liquid at 4°C but solidifies at body temperature]; Sigma Aldrich). Mimics were initially delivered after 3 days, whereas anti-miR and siRNA were delivered immediately after wounding. The treatment was performed every second day for 7 days. Wounds were photographed using an Olympus camera on days 0, 2, 4, 6, and 7 after wounding. Wound areas were calculated as previously described ^{54,55}. To this aim, two perpendicular diameters of the wound were measured by using a Vernier caliper and wound area was calculated using a standard formula for the area of an ellipse (semi-major diameter X semi-minor diameter X Pi). The superficial blood flow of the wounds was sequentially analysed by colour laser Doppler (Colour laser Doppler, Moor, UK), and the ratio of blood flow between the mimic or anti-miR or siRNA treated and the control treated wounds was calculated. Wound size and Doppler analysis was performed in blinded fashion.

### Histology and image analysis

Wounds were harvested, prepared, and analysed essentially as previously described ⁵⁴⁻⁵⁶. At days 5 or 7 post-wounding, wounds were harvested and fixed in 10% buffered formalin (16h at 4°C, Sigma) for embedding in paraffin. Sectioning was done through the wound beyond the midpoint and wound centres as identified by staining with Haematoxylin and Eosin (Sigma). Sections were deparaffinised, rehydrated and stained with rat anti-mouse polyclonal CD31 (Abcam, ab28364, 1:50), α-SMA (Sigma, A2547, 1:100) rabbit anti-mouse FSP-1 (Abcam, ab41532, 1:50), and SLUG (Abcam, ab28364, 1:200); all antibodies overnight at 4°C.

The slides of the staining were examined and photomicrographed in a blinded fashion using Zeiss LSM 780 Confocal Microscope (Zeiss). Vascular density in wounds was counted after staining for CD31 and α-SMA. Ten fields per section/animal (n=8 animal; 400X magnification) were randomly examined and averaged to analyse the number of CD31-positive blood vessels in the wound edges. Images collection was performed manually. Image analysis was performed using Image J software as previously reported in ⁵⁴. Vascular density is expressed per square millimetre. EndMT of ECs was assessed by FSP-1 and SLUG staining combined with CD31 staining. CD31-FSP-1 and CD31-SLUG double positive vessels are expressed as percentage of positive vessels. For analysis of fibrosis: the slides of the staining were examined and photomicrographed in a blinded fashion using a light microscope (Axioscope 2; Zeiss). Six images per slide were captured at 400x magnification for both histological staining. The colour deconvolution ImageJ software was used to evaluate the percentage of red staining which indicate the presence of collagen fibres in the tissue. The morphometric analysis corresponding to the red colours was measured as the percentage of total pixels in each image, using the Threshold (ImageJ software).

### Statistical analysis

Comparisons between different conditions were assessed using 2-tailed Student's t test. Differences among groups were elicited using ANOVA statistical test followed by Bonferroni *post-hoc* analyses as appropriate. Continuous data are expressed as mean ± SD of three independent experiments, each performed in triplicate or quintuplicate, as reported in the legends. P value <0.05 was considered statistically significant. Analyses were performed using GraphPad Prism v5.0.

### miR-148b targets TGFB2 and SMAD2 and regulates EC functions

Gene set enriched analysis (miRpath ²⁴) identified components of the TGF-β pathway as putative targets of miR-148b (Figure 1A). Moreover, the Targetscan algorithm ²⁵ predicted *TGFB2* and *SMAD2* to be direct targets of miR-148b (Figure 2A). Accordingly, both gene targets were predicted to contain a single conserved binding sequence for miR-148b in their 3' untranslated region (UTR) (Figure 2A). To investigate whether miR-148b directly binds the 3'UTR of *TGFB2* and *SMAD2,* the inventors performed a luciferase reporter assay in which luciferase reporter gene was fused to the wild-type 3'UTR of *TGFB2* or *SMAD2,* respectively. Overexpression of miR-148b decreased luciferase activity for each of the putative target genes, whereas mutation of the binding sites restored it (Figure1B and Figure 1B) In addition, miR-148b mimics at the dose of 25nM reduced both target gene mRNA (Figure 2C and Figure 1C and Figure 1D) and protein levels (Figure 2D). Notably, miR-148b upregulation did not affect the expression of *ACVR1* and *ROCK1,* previously identified target genes of miR-148/152 cluster ^{26,27} (Figure 1E). The inventors then investigated the role of miR-148b on the functional properties of ECs. miR-148b overexpression significantly increased the proliferation of human umbilical vein EC (HUVECs), as demonstrated by BrdU incorporation (Figure 2E). The migration capacity of HUVECs transfected with a miR-148b mimic was significantly elevated (Figure 2F), along with their ability to form tubes in an *in vitro* Matrigel assay (Figure 2G).

### miR-148b promotes angiogenesis and accelerates skin wound healing

To follow up these *in vitro* results, the inventors asked whether miR-148b could influence angiogenesis in a mouse model of wound healing. In this model, following skin puncture, the peak of neovascularization occurs between days 4 and 8 after wounding. Vessel density generally returns to initial levels around 3 weeks later. Therefore, before and at the peak of vascularization, this model reports on the angiogenic response ²⁸. Three days after wound injury, the expression of miR-148b is downregulated, going back to basal level after 7 days (Figure 3A). The analysis of Cy3-labelled miR-148b mimics in the wounds demonstrated that the mimics can reach the endothelium *in vivo* (Figure 4A).

Topical delivery of miR-148b mimics starting at 3 days after wounding increased miR-148b expression decreasing the expression levels of both targets at day 7 after wounding (Figure 3B). Moreover, miR-148b overexpression also accelerated wound closure (Figure 3B) and led to a significant increase in wound perfusion detected by Doppler analysis (Figure 3C) and vessels density (Figure 3D). These data indicate that treatment with miR-148b mimics promotes angiogenesis and accelerates wound healing in *vivo.*

### Loss-of-function of miR-148b promotes endothelial- mesenchymal transition

To analyse the phenotypic effect of miR-148b loss-of-function on ECs, HUVECs were transfected with anti-miR-148b oligonucleotides (Figure 1F). The inhibition of miR-148b expression decreased the proliferation of HUVECs (Figure 5A); however, it did not cause a change in the EC migration or tubulogenesis (Figure 5B and 5C). Next, the inventors examined the capacitance (Cm) of the EC membrane, changes in which reflect structural changes in the plasma membrane and rearrangement of the cytoskeleton (the Cm of ECs is ~1.1 µF/cm2, whereas for mesenchymal cells it is ~3.0; µF/cm2)²⁹. Accordingly, the knock-down of miR-148b in ECs increased the membrane capacitance consistent with the occurrence of transition from an endothelial to mesenchymal phenotype (Figure 5D). In line with this, miR-148b loss-of-function increased the expression of its target genes, *TGFB2* and *SMAD2* and the expression of collagen 1A1 *(COL1A1)* and fibroblast specific protein 1 *(FSP-1)* but decreased the expression of *CD31* and *VE-Cadherin,* without detectable changes in *α-SMA or* vimentin *(VIM)* levels (Figure 5E). Moreover, expression analysis of the transcription factors involved in EndMT, such as *SNAIL, SLUG, TWIST and ZEB1*/*2,* showed the strong up-regulation of SLUG after miR-148b inhibition (Figure 5E). Western blot analysis confirmed the upregulation of TGFB2, SMAD2 and its phosphorylation, the appearance of mesenchymal marker COL1A1, FSP-1 along with suppression of CD31 and endothelial VE-cadherin and the up-regulation of SLUG in ECs lacking miR-148b (Figure 5F). Furthermore, immunofluorescence staining revealed that control-treated HUVECs display a typically rounded/cobblestone morphology that was lost in anti-miR-148b treated cells. Anti-miR-148b treatment also resulted in a pronounced decrease in CD31 and VE-cadherin and increase in COL1A1 and FSP-1 immunoreactivity (Figure 5G). Taken together, these data show that loss of miR-148b function leads to EndMT.

### Pro-inflammatory cytokines promote EndMT via downregulation of miR-148b

It has been reported that EndMT is closely linked to inflammation and high levels of pro-inflammatory cytokines ³⁰. To investigate whether cytokine treatment could reduce the expression of miR-148b, thus triggering EndMT, the inventors treated HUVECs with TNF-α, IL-1β, TGFβ1 or TGFβ2, as well as with a combination of cytokines for 6 days (Figure 6A-D). Interestingly, the inventors found that only the combination of TNF-α and IL-1β inhibited miR-148b expression after 3 days, with its strongest downregulation at 6 days (Figure 7A). This was accompanied by significantly increased expression of TGFB2 and SMAD2, at both mRNA and protein levels (Figure 7B and 7C), phosphorylation of SMAD2 (Figure 7C) and increased secretion of TGFβ2 (Figure 8A). To test whether cytokine-induced miR-148b downregulation governs EndMT, the inventors analysed endothelial- and fibroblast-specific markers after the treatment with TNF-α/IL-1β. Consistent with enhancement of EndMT, both mRNA and protein expression of the endothelial markers CD31 and VE-Cadherin were decreased (Figure 7B and 7C). These findings were further confirmed by immunofluorescence staining of HUVECs treated with TNF-α/IL-1β (Figure 7D). In contrast, the mRNA expression and protein levels of fibroblast-specific COL1A1 and FSP-1 (Figure 7B and 7C) were increased 6 days after TNF-α/IL-1β treatment, corroborated by observed increase in cellular and extracellular immunofluorescence signals (Figure 7D). Moreover, upregulation of SLUG was confirmed at mRNA and protein level in cytokine-treated ECs (Figure 7B and 7C). Notably, HUVECs cultured for 14 days in the presence of TNF-α/IL-1β maintained the downregulation of miR-148b and acquired α-SMA in addition to other markers of EndMT (Figure 9A and Figure 9B).

Finally, since let-7b and miR-20a are miRNAs involved in EndMT process^{31, 32}, the inventors have analysed the expression of let-7b and miR-20a showing that while miR-20a and let-7b are both downregulated *in vivo* during wound healing (Figure 10A), only miR-20a is downregulated by cytokine treatment in ECs (Figure 10B).

### miR-148b over-expression rescues cytokine-mediated EndMT through SMAD2

The inventors next examined whether miR-148b gain-of-function could rescue the endothelial phenotype and reduce cytokine-induced EndMT. In response to a miR-148b mimic in HUVECs treated with TNF-α/IL-1β, the inventors observed a decreased of TGFB2, SMAD2, SLUG, FSP-1, COL1A1 and a reduced phosphorylation of SMAD2, whereas the miR-148b mimic rescued CD31 and VE-cadherin at mRNA and protein levels (Figure 8B, Figure 12A and Figure 11A). The maintenance of endothelial markers CD31 and VE-cadherin on ECs membrane and the decrease of COL1A1 and FSP-1 staining was also confirmed by immunostaining (Figure 11B). To better understand the contribution of target genes in this model of EndMT, the inventors interfered with the TGF-β pathway using a TGF-β receptor (ALK5) inhibitor (SB431542) or siRNA for SMAD2. Knockdown of SMAD2 in HUVECs treated with cytokines partially restored *CD31* and *VE-cadherin* expression, whereas blocking of ALK5 reduced the level of SMAD2 phosphorylation but did not rescue endothelial markers expression (Figure 12B and 12C). Then, it was confirmed at protein level that SMAD2 siRNA partially restores the cytokine-induced decrease in expression of CD31 and VE-cadherin, whereas decrease SLUG, FSP-1 and COL1A1 protein expression (Figure 11C). CD31 and VE-cadherin, FSP-1 and COL1A1 results were further confirmed by immunocytochemistry (Figure 11D).

### miR-148b loss-of-function attenuates wound repair by enabling EndMT in a skin wound healing model

Next, the inventors investigated whether miR-148b-mediated EndMT also occurs *in vivo,* in the mouse model of skin wound healing. Delivery of anti-miR-148b, or control oligonucleotides immediately after wounding, and every two days thereafter, resulted in increased expression of *TGFB2* and *SMAD2* target mRNA in the wounds (Figure 14A). Macroscopic analysis showed that wound closure was markedly attenuated in anti-miR-148b-treated wounds (Figure 13A), without effecting wound perfusion and vessel density (Figure 12C and 12D). Not significative improvement in wound closure is detected by SMAD2 siRNA only (Figure 13A and Figure 14B). Then, to investigate EndMT in the wound edge, wounds were stained with CD31 and FSP-1 and SLUG antibodies (Figure 13B and 13C). Confocal analysis showed that, while control-oligonucleotide-treated wounds contained only CD31-positive vessels, anti-miR-148b-treated wounds were comprised of CD31-FSP-1 or CD31-SLUG double-positive vessels, consistent with the occurrence of EndMT (Figure 13D). Moreover, anti-miR-148b-treated wounds showed an increase in the total number of FSP-1 positive cells compared with the control oligonucleotide-treated wounds (Figure 13D) whereas staining with PicroSirius Red did not detect any increase of collagen deposition in the wounds (Figure 14E). Finally, to confirm that loss of miR-148b-mediated EndMT occurs through SMAD2 regulation, the inventors knocked down SMAD2 by siRNA *in vivo* in the anti-miR-148b treated wounds (Figure 14B). Knocking down SMAD2 in this model, promotes wound closure (Figure 13A), without significantly promoting wound perfusion and vessel density (Figure 14C and 14D). Moreover, SMAD2 siRNA reduced the percentage of vessels double positive for CD31-FSP-1 and CD31-SLUG and decreased the total number of FSP-1 positive cells in the wounds treated with anti-miR-148b (Figure 13D). Overall, these results show that inhibition of miR-148b *in vivo* impaired wound healing and promoted EndMT on the wound edge via SMAD2 upregulation.

### miR-148b is downregulated in diabetic wounds

6- to 7-week-old male C57BL/6J mice (n=5 per group) were made diabetic using streptozotocin (STZ) or left normoglycemic after STZ buffer. At either 1 or 3 months of diabetes, a full-thickness wound was created in the dorsal skin using a sterile 5-mm-wide biopsy punch. After 3 days from post-wound, mice were sacrificed by an overdose of anaesthetic and the wounds and surrounding skin were removed. RNA was extracted from wounds using the FastPrep tissue lyser (MPbio) and miRNeasy kits (Qiagen). miR-148b was measured in the samples by quantitative PCR showing a significant down regulation of miR-148b in the wounds from diabetic mice (1.2+/- 0.25 vs 0.15 +/-0.09 T-test p=0.0023).

### Discussion

Here the inventors show for the first time that miR-148b has a crucial role in determining EC function and plasticity by regulating the TGF-β pathway. Overexpression of miR-148b enhances EC proliferation, migration and *in vitro* angiogenesis via targeting *TGFB2* and *SMAD2.* The results demonstrate that miR-148b is of particular utility in clinical applications relating to conditions in which EndMT is involved. For example, miRNA -148b may be used to treat conditions where it is desirable to inhibit EndMT, such as in the treatment of dermal wounds and/or chronic wounds, such as dermal wounds in diabetic patients. *In vivo* delivery of miR-148b to injured skin promotes angiogenesis and accelerates wound closure. In contrast, miR-148b inhibition promotes the acquisition of a mesenchymal phenotype by ECs via upregulation of *TGFB2* and *SMAD2.* This is associated with poor *in vivo* skin wound closure and activation of EndMT in dermal vessels.

One of the first indications of the involvement of miRNAs in EndMT came from Ghosh *et al.* ³³, who reported differential expression of specific set of miRNAs during transition of mouse cardiac ECs to a mesenchymal phenotype. Since then miRNAs have been linked to regulation of EndMT through different mechanisms. *In vitro* and *in vivo* studies demonstrated that decreased FGF signalling leads to reduction in let-7 expression and initiation of TGFβ-dependent Endo-MT ³¹. Recently, miR-20a was shown to target multiple genes in TGF-β signalling such as ALK5, TGFβR2 and SARA, thereby negatively regulating EndMT. The expression of miR-20a, which is normally downregulated during EndMT, is rescued by FGF2 ³². In the inventors' study, both miR-20a and let-7b are downregulated *in vivo* in the model of wound healing, whereas only miR-20a is also downregulated by cytokine treatment in HUVECs. Interestingly, further bioinformatic analysis demonstrated that the three miRNAs target a different set of genes of the TGF-β pathway with minimal (2 on 20 genes between miR-148b and let-7b) or no overlapping (between miR-148b and miR-20a targets). Therefore, miR-20a, let-7b and miR-148b can interfere at different level of the TGF-β pathway showing a possible synergic effect during skin wound healing.

The occurrence of EndMT postnatally has been associated with different types of fibrotic disease such as that in kidney ¹² and heart ³⁴, both of which are associated with excessive collagen expression and deposition. Previous studies reported the importance of EndMT in dermal fibrosis, showing that delay in wound closure is associated with excessive collagen deposition ³¹. In the present study induction of EndMT was also found to have a detrimental influence on skin wound healing. Following delivery of anti-miR-148b to the wounds, EndMT accounted for around 20% of skin vessels that demonstrated mesenchymal transition. This is highly important as, so transformed, these cells can be expected to secrete large amounts of collagen and other ECM proteins, which could contribute to fibrosis. In line with this, in the inventors' model the inventors detected an increase in the recruitment of FSP-1 positive cells in the wounds treated with anti-miR-148b. However, analysis with PicroSirius Red did not show any accumulation of collagen in the wounds treated with anti-miR-148b at day 7 after wounding.

EndMT may lead to endothelial cells acquiring a variety of different mesenchymal fates through different stage of differentiation. The early endothelial response is characterized by a partial downregulation of endothelial markers, junction dismantling and up-regulation of some early mesenchymal markers. At later times, expression of endothelial markers further declines while more mesenchymal markers including matrix proteins are up-regulated ⁴. An abnormally increased inflammatory environment is often linked with phenotypic changes in ECs ³⁶. Chronic treatment of ECs with a combination of TNF-α and IL-1β over 6 days decreased the expression of miR-148b in ECs while it increased the expression and secretion of TGFβ2. In addition, the acquisition of a mesenchymal phenotype due to inflammation by dermal ECs is likely to lead to endothelial dysfunction, seen through decreased EC migration or dysregulation of inflammatory cell recruitment ³⁷.

Inflammation is sufficient to induce all events required for EndMT except for *de novo* acquisition of α-SMA, which is strictly dependent on TGF-β ³⁰. In the inventors' experiments, cytokine treatment or miR-148b inhibition increase the release of TGFβ2, as a direct target, which drives the EndMT process, perhaps partly or incompletely at least after 6 days. Consequently, EndMT appeared completed upon 14 days of TNF-α/IL-1β exposure. By this point ECs have acquired α-SMA in addition to other markers of EndMT.

Recent reports show that inflammatory cytokines such as TNF-α and IL1-β can also trigger EndMT via binding to their receptors and by inducing NF-kB translocation to the nucleus, driving the expression of EndMT-specific genes ^{30, 38}. Such inflammatory signals facilitate EndMT by increasing endogenous TGF-β expression in an NFκB-dependent manner, creating a feed forward signalling mechanism ³⁹. Interestingly, the treatment with the miR-148b mimics has inhibited EndMT progression in cytokine-treated ECs. In agreement with this, a recent study demonstrated that pro-inflammatory molecules present in the serum of patients with Kawasaki syndrome induce EndMT through downregulation of miR-483 in ECs, whereas the overexpression of miR-483 using mimic oligonucleotides suppresses EndMT and restores EC function ⁴⁰.

In the inventors' *in vitro* model, the mechanism is based on activation of SMAD2 as demonstrated by rescue of EndMT using SMAD2 antisense. Alternatively, the inventors have used SB43152, a specific inhibitor of ALK5, at the dose of 10µM to inhibit SMAD2 phosphorylation and inhibit EndMT progression. However, SB43152 did not restore endothelial markers as showed by miR-148b mimics and SMAD2 siRNA. Therefore, miR-148b mimic or SMAD2 siRNA reduced SMAD2 phosphorylation completely through SMAD2 mRNA degradation. Thus, explaining the discrepancy in results between the two treatments. It is worthy to note that another possible layer of the complex regulation of SMAD2 phosphorylation is through the mitotic kinase Mps1 which phosphorylate SMAD2 independently of type I receptors ⁴¹.

In the *in vivo* model of wound healing, SMAD2 siRNA rescued anti-miR-148b-mediated EndMT and increase the wound closure rate at day 7; however, at the same time point SMAD2 siRNA alone had not effect on wound closure rate. The discrepancy of these results could be explained that during wound healing other SMAD-independent pathway are regulated, affecting wound closure and EndMT. For example, TGF-β and inflammatory cytokine have been shown to activate diverse non-SMAD parallel downstream pathways, such as extra-cellular signal-regulated kinase (ERK), c-Jun NH2-terminal kinase (JNK), p38 MAP kinase ⁴². Moreover, a recent study analysed the impact of EndMT on the skin wounds showed the existence of a Notch-mediated EndMT during skin wound healing ⁴³.

The participation of EndMT during wound healing, the demonstration that miR-148b inhibits EndMT, and the demonstration that in diabetic wounds, levels of miRNA-148b are low, enables the use of miR-148b and miR-148b mimics in the treatment of patients with wounds, such as dermal wounds and/or chronic or slow- healing wounds, such as diabetic non-healing wounds. Furthermore, identification of the source of TGF-β and other active ligands that can initiate EndMT, and increased understanding the molecular mechanisms that regulate EndMT, will be of great value in providing cellular targets amenable to therapeutic intervention for fibrotic disease. In the inventors' experience, the skin wound healing model is an excellent model to assess the potential impact of EndMT on the vessels that have formed during the wound healing process. The excisional wounds rapidly close, with the active tissue remodelling of the wound area occurring in reproducible way at specific time-points. The use of endothelial lineage tracing mouse model during wound healing ⁴³ has further corroborated the inventors' data and highlighted the critical role of EndMT during skin wound healing.

The TGF-β signalling pathway is considered a promising target for the treatment of many diseases, including pathological skin conditions. Most of the component of the TGF-β pathway has been targeted for drug development through numerous design strategies. Several have been developed through preclinical to clinical trials, such as oligonucleotide antisense ⁴⁴. Indeed, TGF-β antisense oligonucleotides reduced scarring and improved surgical outcome in animal models ⁴⁵. Moreover, Smad3 antisense oligonucleotides accelerated wound healing and reduced scarring in a mouse excisional wound model ⁴¹. In this context, the benefit of miRNA-based therapy is the possibility of interfering with the expressional regulation of multiple genes. The inventors have demonstrated that miR-148b mimics impacts on different components of TGF-β pathway, thus amplifying its therapeutic potential in a model of skin wound healing.

Moreover, an important factor that the inventors have considered in the inventors' *in vivo* experiments is also at which stage of the wound healing process the treatment with miR-148b mimics could be beneficial. Indeed, several studies reported that reducing TGF-β pathway at later stage during skin wound healing improved scarring outcome ⁴⁸. The inventors have then treated the skin wounds at 3 days from the wounding, when expression of miR-148b decreased, avoiding to inhibits TGF-β signalling during the inflammatory phase, where TGF-β is necessary to start the healing process ⁴⁹.

In summary, the data presented here identify miR-148b as a novel regulator of TGF-β signalling and EC plasticity and support its use as a therapeutic target for promotion of tissue repair and treatment of diseases or conditions in which EndMT is enhanced, such as in dermal and/or slow healing or chronic wounds.

### References

1. Goumans, MJ, and Ten Dijke, P (2017). TGF-beta Signaling in Control of Cardiovascular Function. Cold Spring Harbor perspectives in biology*.*
2. van Meeteren, LA, and ten Dijke, P (2012). Regulation of endothelial cell plasticity by TGF-beta. Cell and tissue research 347: 177-186.
3. Akhurst, RJ (2012). The paradoxical TGF-beta vasculopathies. Nature genetics 44: 838-839.
4. Dejana, E, Hirschi, KK, and Simons, M (2017). The molecular basis of endothelial cell plasticity. Nat Commun 8: 14361.
5. Piera-Velazquez, S, Mendoza, FA, and Jimenez, SA (2016). Endothelial to Mesenchymal Transition (EndoMT) in the Pathogenesis of Human Fibrotic Diseases. Journal of clinical medicine 5***.***
6. Kovacic, JC, Mercader, N, Torres, M, Boehm, M, and Fuster, V (2012). Epithelial-to-mesenchymal and endothelial-to-mesenchymal transition: from cardiovascular development to disease. Circulation 125: 1795-1808.
7. Piera-Velazquez, S, and Jimenez, SA (2012). Molecular mechanisms of endothelial to mesenchymal cell transition (EndoMT) in experimentally induced fibrotic diseases. Fibrogenesis Tissue Repair 5: S7.
8. Arciniegas, E, Neves, CY, Carrillo, LM, Zambrano, EA, and Ramirez, R (2005). Endothelial-mesenchymal transition occurs during embryonic pulmonary artery development. Endothelium 12: 193-200.
9. Armstrong, EJ, and Bischoff, J (2004). Heart valve development: endothelial cell signaling and differentiation. Circ Res 95: 459-470.
10. Zeisberg, EM, Potenta, S, Xie, L, Zeisberg, M, and Kalluri, R (2007). Discovery of endothelial to mesenchymal transition as a source for carcinoma-associated fibroblasts. Cancer Res 67: 10123-10128.
11. Zeisberg, EM, Tarnavski, O, Zeisberg, M, Dorfman, AL, McMullen, JR, Gustafsson, E, et al. (2007). Endothelial-to-mesenchymal transition contributes to cardiac fibrosis. Nat Med 13: 952-961.
12. Zeisberg, EM, Potenta, SE, Sugimoto, H, Zeisberg, M, and Kalluri, R (2008). Fibroblasts in kidney fibrosis emerge via endothelial-to-mesenchymal transition. Journal of the American Society of Nephrology : JASN 19: 2282-2287.
13. Cooley, BC, Nevado, J, Mellad, J, Yang, D, St Hilaire, C, Negro, A, et al. (2014). TGF-beta signaling mediates endothelial-to-mesenchymal transition (EndMT) during vein graft remodeling. Sci Transl Med 6: 227ra234.
14. Evrard, SM, Lecce, L, Michelis, KC, Nomura-Kitabayashi, A, Pandey, G, Purushothaman, KR, et al. (2016). Endothelial to mesenchymal transition is common in atherosclerotic lesions and is associated with plaque instability. Nat Commun 7: 11853.
15. Ha, M, and Kim, VN (2014). Regulation of microRNA biogenesis. Nature reviews Molecular cell biology 15: 509-524.
16. Li, Z, and Rana, TM (2014). Therapeutic targeting of microRNAs: current status and future challenges. Nat Rev Drug Discov 13: 622-638.
17. Caporali, A, and Emanueli, C (2011). MicroRNA regulation in angiogenesis. Vascul Pharmacol 55: 79-86.
18. Caporali, A, and Emanueli, C (2012). MicroRNAs in Postischemic Vascular Repair. Cardiol Res Pract 2012: 486702.
19. Kurakula, K, Goumans, MJ, and Ten Dijke, P (2015). Regulatory RNAs controlling vascular (dys)function by affecting TGF-ss family signalling. EXCLI journal 14: 832-850.
20. Ghosh, AK, Nagpal, V, Covington, JW, Michaels, MA, and Vaughan, DE (2012). Molecular basis of cardiac endothelial-to-mesenchymal transition (EndMT): differential expression of microRNAs during EndMT. Cellular signalling 24: 1031-1036.
21. Chen, Y, Song, YX, and Wang, ZN (2013). The microRNA-148/152 family: multi-faceted players. Molecular cancer 12: 43.
22. Serino, G, Sallustio, F, Cox, SN, Pesce, F, and Schena, FP (2012). Abnormal miR-148b expression promotes aberrant glycosylation of IgA1 in IgA nephropathy. Journal of the American Society of Nephrology : JASN 23: 814-824.
23. Bidzhekov, K, Gan, L, Denecke, B, Rostalsky, A, Hristov, M, Koeppel, TA, et al. (2012). microRNA expression signatures and parallels between monocyte subsets and atherosclerotic plaque in humans. Thrombosis and haemostasis 107: 619-625.
24. Vlachos, IS, Zagganas, K, Paraskevopoulou, MD, Georgakilas, G, Karagkouni, D, Vergoulis, T, et al. (2015). DIANA-miRPath v3.0: deciphering microRNA function with experimental support. Nucleic Acids Res 43: W460-466.
25. Grimson, A, Farh, KK, Johnston, WK, Garrett-Engele, P, Lim, LP, and Bartel, DP (2007). MicroRNA targeting specificity in mammals: determinants beyond seed pairing. Mol Cell 27: 91-105.
26. Song, H, Wang, Q, Wen, J, Liu, S, Gao, X, Cheng, J, et al. (2012). ACVR1, a therapeutic target of fibrodysplasia ossificans progressiva, is negatively regulated by miR-148a. Int J Mol Sci 13: 2063-2077.
27. Zhang, J, Ying, ZZ, Tang, ZL, Long, LQ, and Li, K (2012). MicroRNA-148a promotes myogenic differentiation by targeting the ROCK1 gene. J Biol Chem 287: 21093-21101.
28. Simons, M, Alitalo, K, Annex, BH, Augustin, HG, Beam, C, Berk, BC, et al. (2015). State-of-the-Art Methods for Evaluation of Angiogenesis and Tissue Vascularization: A Scientific Statement From the American Heart Association. Circ Res 116: e99-132.
29. Giaever, I, and Keese, CR (1991). Micromotion of mammalian cells measured electrically. Proc Natl Acad Sci U S A 88: 7896-7900.
30. Rieder, F, Kessler, SP, West, GA, Bhilocha, S, de la Motte, C, Sadler, TM, et al. (2011). Inflammation-induced endothelial-to-mesenchymal transition: a novel mechanism of intestinal fibrosis. Am J Pathol 179: 2660-2673.
31. Chen, PY, Qin, L, Barnes, C, Charisse, K, Yi, T, Zhang, X, et al. (2012). FGF regulates TGF-beta signaling and endothelial-to-mesenchymal transition via control of let-7 miRNA expression. Cell Rep 2: 1684-1696.
32. Correia, AC, Moonen, JR, Brinker, MG, and Krenning, G (2016). FGF2 inhibits endothelial-mesenchymal transition through microRNA-20a-mediated repression of canonical TGF-beta signaling. J Cell Sci 129: 569-579.
33. Ghosh, AK, Nagpal, V, Covington, JW, Michaels, MA, and Vaughan, DE (2012). Molecular basis of cardiac endothelial-to-mesenchymal transition (EndMT): Differential expression of microRNAs during EndMT. Cellular Signalling 24: 1031-1036.
34. Zeisberg, EM, Tarnavski, O, Zeisberg, M, Dorfman, AL, McMullen, JR, Gustafsson, E, et al. (2007). Endothelial-to-mesenchymal transition contributes to cardiac fibrosis. Nat Med 13: 952-961.
35. Manetti, M, Romano, E, Rosa, I, Guiducci, S, Bellando-Randone, S, De Paulis, A, et al. (2017). Endothelial-to-mesenchymal transition contributes to endothelial dysfunction and dermal fibrosis in systemic sclerosis. Annals of the Rheumatic Diseases*.*
36. Romero, LI, Zhang, DN, Herron, GS, and Karasek, MA (1997). Interleukin-1 induces major phenotypic changes in human skin microvascular endothelial cells. Journal of cellular physiology 173: 84-92.
37. Manetti, M, Romano, E, Rosa, I, Guiducci, S, Bellando-Randone, S, De Paulis, A, et al. (2017). Endothelial-to-mesenchymal transition contributes to endothelial dysfunction and dermal fibrosis in systemic sclerosis. Ann Rheum Dis 76: 924-934.
38. Mahler, GJ, Farrar, EJ, and Butcher, JT (2013). Inflammatory cytokines promote mesenchymal transformation in embryonic and adult valve endothelial cells. Arterioscler Thromb Vasc Biol 33: 121-130.
39. Liu, RM, and Gaston Pravia, KA (2010). Oxidative stress and glutathione in TGF-beta-mediated fibrogenesis. Free radical biology & medicine 48: 1-15.
40. He, M, Chen, Z, Martin, M, Zhang, J, Sangwung, P, Woo, B, et al. (2017). miR-483 Targeting of CTGF Suppresses Endothelial-to-Mesenchymal Transition: Therapeutic Implications in Kawasaki Disease. Circ Res 120: 354-365.
41. Zhu, S, Wang, W, Clarke, DC, and Liu, X (2007). Activation of Mps1 promotes transforming growth factor-beta-independent Smad signaling. J Biol Chem 282: 18327-18338.
42. Derynck, R, and Zhang, YE (2003). Smad-dependent and Smad-independent pathways in TGF-beta family signalling. Nature 425: 577-584.
43. Patel, J, Baz, B, Wong, HY, Lee, JS, and Khosrotehrani, K (2017). Accelerated Endothelial to Mesenchymal Transition increased fibrosis via deleting Notch signalling in wound vasculature.
44. Akhurst, RJ, and Hata, A (2012). Targeting the TGFbeta signalling pathway in disease. Nature reviews Drug discovery 11: 790-811.
45. Choi, BM, Kwak, HJ, Jun, CD, Park, SD, Kim, KY, Kim, HR, et al. (1996). Control of scarring in adult wounds using antisense transforming growth factor-beta 1 oligodeoxynucleotides. Immunology and cell biology 74: 144-150.
46. Cordeiro, MF, Mead, A, Ali, RR, Alexander, RA, Murray, S, Chen, C, et al. (2003). Novel antisense oligonucleotides targeting TGF-beta inhibit in vivo scarring and improve surgical outcome. Gene therapy 10: 59-71.
47. Mulholland, EJ, Dunne, N, and McCarthy, HO (2017). MicroRNA as Therapeutic Targets for Chronic Wound Healing. Molecular therapy Nucleic acids 8: 46-55.
48. Finnson, KW, McLean, S, Di Guglielmo, GM, and Philip, A (2013). Dynamics of Transforming Growth Factor Beta Signaling in Wound Healing and Scarring. Advances in wound care 2: 195-214.
49. Finnson, KW, Arany, PR, and Philip, A (2013). Transforming Growth Factor Beta Signaling in Cutaneous Wound Healing: Lessons Learned from Animal Studies. Advances in wound care 2: 225-237.
50. Agarwal, V, Bell, GW, Nam, JW, and Bartel, DP (2015). Predicting effective microRNA target sites in mammalian mRNAs. Elife 4**.**
51. Friedman, RC, Farh, KK, Burge, CB, and Bartel, DP (2009). Most mammalian mRNAs are conserved targets of microRNAs. Genome Res 19: 92-105.
52. Schmittgen, TD, and Livak, KJ (2008). Analyzing real-time PCR data by the comparative C(T) method. Nature protocols 3: 1101-1108.
53. Caporali, A, Meloni, M, Vollenkle, C, Bonci, D, Sala-Newby, GB, Addis, R, et al. (2011). Deregulation of microRNA-503 contributes to diabetes mellitus-induced impairment of endothelial function and reparative angiogenesis after limb ischemia. Circulation 123: 282-291.
54. Al Haj Zen, A, Nawrot, DA, Howarth, A, Caporali, A, Ebner, D, Vernet, A, et al. (2016). The Retinoid Agonist Tazarotene Promotes Angiogenesis and Wound Healing. Mol Ther 24: 1745-1759.
55. Caporali, A, Meloni, M, Miller, AM, Vierlinger, K, Cardinali, A, Spinetti, G, et al. (2012). Soluble ST2 is regulated by p75 neurotrophin receptor and predicts mortality in diabetic patients with critical limb ischemia. Arteriosclerosis, thrombosis, and vascular biology 32: e149-160.
56. Barcelos, LS, Duplaa, C, Krankel, N, Graiani, G, Invernici, G, Katare, R, et al. (2009). Human CD133+ progenitor cells promote the healing of diabetic ischemic ulcers by paracrine stimulation of angiogenesis and activation of Wnt signaling. Circ Res 104: 1095-1102.

## Claims

1. A miRNA-148b for use in the treatment of a dermal wound.

2. The miRNA-148b for use according to claim 1, wherein said wound is a diabetic wound, for example a diabetic leg or foot ulcer.

3. The miRNA-148b for use according to claim 1 or claim 2wherein said treatment comprises administration of microRNA-148b (miR-148b).

4. The miRNA-148b for use for use according to claim 3, wherein said miR-148b comprises the nucleotide sequence shown as SEQ ID NO: 2 or SEQ ID NO:3.

5. The miRNA-148b for use according to claim 3 or claim 4, wherein the miR-148b comprises the seed sequence of the nucleotide sequence shown as SEQ ID NO: 4 or SEQ ID NO:5.

6. The miRNA-148b for use according to any one of claims 3 to 5, wherein the miR-148b is a miR-148b mimic, wherein said miR-148b mimic which has a biologically equivalent effect to a naturally derived miRNA 148-b with respect to one or more one or more of the ability to reduce expression of *TGFB2* and/or *SMAD2,* , to accelerate wound closure, to increase wound perfusion and/or to increase vessel density in a wound.

7. The miRNA-148b for use according to any one of claims 3 to 5 wherein the miR-148b comprises a pri- miR-148b, a pre- miR-148b or a mature single strand miR-148b.

8. The miRNA-148b for use according to any one of claims 3 to 7, wherein the miR-148b comprises the nucleotide sequence shown as SEQ ID NO: 1.

9. The miRNA-148b for use according to any one of claims 3 to 5, wherein the miR-148b is a functional fragment of a of a full-length miR-148b, which shares the ability of the full length miR-148b to accelerate angiogenesis in a wound and/or accelerate wound closure.

10. The miRNA-148b for use according to any one of claims 1 to 8 wherein said miRNA-148b is comprised within a vector.

11. An in vitro method of promoting transition of endothelial cells to a mesenchymal phenotype, said method comprising administration of an inhibitor of miR-148b to said endothelial cells, wherein said inhibitor is selected from the group consisting of a LNA anti-miR-148b-3p, synthetic 'Tough Decoy' (TuD) molecule against miR-148b-3p, and a miR-148b-3p sponge.

12. An in vitro method of enhancing collagen production in a cell, population of cells, or tissue, said method comprising the administration of a miR-148b inhibitor to said cell, population of cells, or tissue, wherein said inhibitor is is selected from the group consisting of a LNA anti-miR-148b-3p, synthetic 'Tough Decoy' (TuD) molecule against miR-148b-3p, and a miR-148b-3p sponge.

## Patentansprüche

1. Eine miRNA-148b zur Verwendung bei der Behandlung einer Hautwunde.

2. miRNA-148b zur Verwendung gemäß Anspruch 1, wobei die Wunde eine diabetische Wunde ist, zum Beispiel ein diabetisches Bein- oder Fußgeschwür.

3. miRNA-148b zur Verwendung gemäß Anspruch 1 oder Anspruch 2, wobei die Behandlung die Verabreichung von microRNA-148b (miR-148b) beinhaltet.

4. miRNA-148b zur Verwendung gemäß Anspruch 3, wobei die miR-148b die als SEQ ID NO: 2 oder SEQ ID NO: 3 gezeigte Nukleotidsequenz beinhaltet.

5. miRNA-148b zur Verwendung gemäß Anspruch 3 oder Anspruch 4, wobei die miR-148b die Seed-Sequenz der als SEQ ID NO: 4 oder SEQ ID NO: 5 gezeigten Nukleotidsequenz beinhaltet.

6. miRNA-148b zur Verwendung gemäß einem der Ansprüche 3 bis 5, wobei die miR-148b ein miR-148b-Mimetikum ist, wobei das miR-148b-Mimetikum eine biologisch äquivalente Wirkung zu einer natürlich abgeleiteten miRNA 148-b in Bezug auf eine oder mehrere der Fähigkeit aufweist, die Expression von *TGFB2* und/oder *SMAD2* zu reduzieren, den Wundverschluss zu beschleunigen, die Wundperfusion zu erhöhen und/oder die Gefäßdichte in einer Wunde zu erhöhen.

7. miRNA-148b zur Verwendung gemäß einem der Ansprüche 3 bis 5, wobei die miR-148b eine pri-miR-148b, eine pre-miR-148b oder eine reife einzelsträngige miR-148b beinhaltet.

8. miRNA-148b zur Verwendung gemäß einem der Ansprüche 3 bis 7, wobei die miR-148b die als SEQ ID NO: 1 gezeigte Nukleotidsequenz beinhaltet.

9. miRNA-148b zur Verwendung gemäß einem der Ansprüche 3 bis 5, wobei die miR-148b ein funktionelles Fragment eines Volllängen-miR-148b ist, das die Fähigkeit der Volllängen-miR-148b teilt, die Angiogenese in einer Wunde zu beschleunigen und/oder den Wundverschluss zu beschleunigen.

10. miRNA-148b zur Verwendung gemäß einem der Ansprüche 1 bis 8, wobei die miRNA-148b in einem Vektor enthalten ist.

11. Ein In-vitro-Verfahren zur Förderung des Übergangs von Endothelzellen zu einem mesenchymalen Phänotyp, wobei das Verfahren die Verabreichung eines Inhibitors von miR-148b an die Endothelzellen beinhaltet, wobei der Inhibitor ausgewählt ist aus der Gruppe, die aus einem LNA-Anti-miR-148b-3p-, synthetischen "Tough Decoy"(TuD)-Molekül gegen miR-148b-3p und einem miR-148b-3p-Schwamm besteht.

12. Ein In-vitro-Verfahren zur Verbesserung der Kollagenproduktion in einer Zelle, einer Population von Zellen oder einem Gewebe, wobei das Verfahren die Verabreichung eines miR-148b-Inhibitors an die Zelle, die Population von Zellen oder das Gewebe beinhaltet, wobei der Inhibitor ausgewählt ist aus der Gruppe, die aus einem LNA-Anti-miR-148b-3p-, synthetischen "Tough Decoy"(TuD)-Molekül gegen miR-148b-3p und einem miR-148b-3p-Schwamm besteht.

## Revendications

1. Un miARN-148b destiné à être utilisé dans le traitement d'une plaie dermique.

2. Le miARN-148b destiné à être utilisé selon la revendication 1, dans lequel ladite plaie est une plaie diabétique, par exemple un ulcère de jambe ou de pied diabétique.

3. Le miARN-148b destiné à être utilisé selon la revendication 1 ou la revendication 2 dans lequel ledit traitement comprend l'administration de microARN-148b (miR-148b).

4. Le miARN-148b destiné à être utilisé selon la revendication 3, ledit miR-148b comprenant la séquence nucléotidique représentée par SEQ ID NO : 2 ou SEQ ID NO : 3.

5. Le miARN-148b destiné à être utilisé selon la revendication 3 ou la revendication 4, le miR-148b comprenant la séquence graine de la séquence nucléotidique représentée par SEQ ID NO : 4 ou SEQ ID NO : 5.

6. Le miARN-148b destiné à être utilisé selon l'une quelconque des revendications 3 à 5, le miR-148b étant un mimétique de miR-148b, ledit mimétique de miR-148b ayant un effet biologiquement équivalent à celui d'un miARN 148-b dérivé naturellement en ce qui concerne une ou plusieurs capacités parmi la capacité à réduire l'expression de *TGFB2* et/ou de *SMAD2,* à accélérer la fermeture de plaie, à augmenter la perfusion de plaie et/ou à augmenter la densité de vaisseaux dans une plaie.

7. Le miARN-148b destiné à être utilisé selon l'une quelconque des revendications 3 à 5, le miR-148b comprenant un pri-miR-148b, un pré-miR-148b ou un miR-148b mature simple brin.

8. Le miARN-148b destiné à être utilisé selon l'une quelconque des revendications 3 à 7, le miR-148b comprenant la séquence nucléotidique représentée par SEQ ID NO : 1.

9. Le miARN-148b destiné à être utilisé selon l'une quelconque des revendications 3 à 5, le miR-148b étant un fragment fonctionnel d'un miR-148b de pleine longueur, qui partage la capacité du miR-148b de pleine longueur à accélérer l'angiogenèse dans une plaie et/ou à accélérer la fermeture de plaie.

10. Le miARN-148b destiné à être utilisé selon l'une quelconque des revendications 1 à 8, ledit miARN-148b étant compris au sein d'un vecteur.

11. Un procédé *in vitro* de promotion de la transition de cellules endothéliales vers un phénotype mésenchymateux, ledit procédé comprenant l'administration d'un inhibiteur de miR-148b auxdites cellules endothéliales, ledit inhibiteur étant sélectionné dans le groupe constitué d'un LNA anti-miR-148b-3p, une molécule synthétique « leurre dur » (TuD) contre miR-148b-3p, et d'une éponge à miR-148b-3p.

12. Un procédé *in vitro* d'amélioration de la production de collagène dans une cellule, une population de cellules, ou un tissu, ledit procédé comprenant l'administration d'un inhibiteur de miR-148b à ladite cellule, population de cellules, ou tissu, ledit inhibiteur étant sélectionné dans le groupe constitué d'un LNA anti-miR-148b-3p, une molécule synthétique « leurre dur » (TuD) contre miR-148b-3p, et d'une éponge à miR-148b-3p.
